# EUROPEAN PATENT APPLICATION

(11) **EP 4 679 075 A2**
(43) Date of publication of application: **14.01.2026**
(21) Application number: 25219875.9
(22) Date of filing: 13.05.2021
(51) Int. Cl.: G01N 27/327

(54) **ANALYTE SENSORS FEATURING ONE OR MORE DETECTION-FACILITATING ENHANCEMENTS**

(30) Priority: 10.06.2020 US 202063037051 P
(62) Divisional of application: 21756087.9
(71) Applicant: Abbott Diabetes Care Inc., Alameda, CA 94502 (US)
(72) Inventor: LIU, Zenghe, Alameda, 94502 (US); FOX, Cade Brylee, Alameda, 94502 (US); MCCANLESS, Jonathan D., Alameda, 94502 (US); FELDMAN, Benjamin J., Alameda, 94502 (US)
(74) Representative: Mathys & Squire

(57) **Abstract**

Analyte sensors are being increasingly employed for monitoring various analytes in vivo. Analyte sensors configured to monitor multiple analytes are also in development. Sufficient sensitivity for low-abundance analytes and multiple analytes having differing membrane permeability values may complicate analyte detection in some cases. Analyte sensors may feature enhancements to address one or both of these issues. Some analyte sensors may comprise a carbon working electrode comprising a dielectric substrate, one or more apertures extending through the dielectric substrate and filled with a carbon conductor pillar, a carbon conductor coating on a first face of the dielectric substrate in direct contact with each carbon conductor pillar, and one or more active areas on a second face of the dielectric substrate in electrical communication with the carbon conductor pillars. Photopolymerized mass transport limiting membranes may be used in combination with such carbon working electrodes or with other working electrode types.

## Description

### BACKGROUND

The detection of various analytes within an individual can sometimes be vital for monitoring the condition of their health. Deviation from normal analyte levels can often be indicative of a number of physiological conditions. Glucose levels, for example, can be particularly important to detect and monitor in diabetic individuals. By monitoring glucose levels with sufficient regularity, a diabetic individual may be able to take corrective action (*e*.*g*., by injecting insulin to lower glucose levels or by eating to raise glucose levels) before significant physiological harm occurs. Monitoring of other analytes may be desirable for other various physiological conditions. Monitoring of multiple analytes may also be desirable in some instances, particularly for comorbid conditions resulting in simultaneous dysregulation of two or more analytes in combination with one another.

Many analytes represent intriguing targets for physiological analyses, provided that a suitable detection chemistry can be identified. To this end, *in vivo* analyte sensors configured for assaying various physiological analytes have been developed and refined over recent years, many of which utilize enzyme-based detection strategies to facilitate detection specificity. Indeed, *in vivo* analyte sensors utilizing a glucose-responsive enzyme for monitoring blood glucose levels are now in common use among diabetic individuals. *In vivo* analyte sensors for other analytes are in various stages of development, including *in vivo* analyte sensors capable of monitoring multiple analytes. Poor sensitivity for low-abundance analytes may be especially problematic for some analyte sensors, particularly due to excessive background signal arising from interaction of an interferent with a carbon working electrode.

To improve biocompatibility, *in vivo* analyte sensors may include a membrane disposed over the implanted portion of the sensor, particularly a membrane that overcoats at least the active area(s) of the analyte sensor. In addition to promoting biocompatibility, the membrane may be permeable or semipermeable to an analyte of interest and limit the overall flux of the analyte to the active area(s) of the analyte sensor. Such mass transport limiting membranes may aid in avoiding overload (saturation) of the sensing components within the active area(s), thereby improving sensor performance and accuracy. By limiting mass transport of an analyte, the chemical kinetics of the sensing process may become analyte-limited rather than enzyme-limited, thereby allowing the sensor output to be correlated readily with the amount of analyte present.

Another difficulty associated with analyte monitoring is that various analytes may exhibit differential permeability through a given mass transport limiting membrane. The most common mass transport limiting membranes employed in *in vivo* analyte sensors are polyvinylpyridine or polyvinylimidazole polymers and copolymers, which may be readily applied to a working electrode by dip coating techniques. Polyurethane is also commonly used to form mass transport limiting membranes. Among this limited suite of polymers, it can sometimes be difficult to identify a suitable membrane chemistry for facilitating satisfactory permeability of a particular analyte. The issue of differential analyte permeability may become even more complex in analyte sensors configured for assaying multiple analytes, wherein compositionally distinct mass transport limiting membranes may be needed upon different portions of a sensor tail to afford satisfactory permeability and/or a stable response for each analyte. Differing membrane permeability values may lead to vastly different response sensitivities between two analytes, which may complicate detection. Dip-coating processes for applying a mass transport limiting membrane having compositionally distinct portions upon an analyte sensor tail are feasible but may significantly increase manufacturing complexity. Moreover, membrane chemistries other than those based upon polyvinylpyridine or polyvinylimidazole may not be readily extendible to dip coating processes. Differing sensitivities for multiple analytes may sometimes be partially compensated for by using active areas of different sizes (*e.g.,* smaller active areas for analytes having high sensitivity/permeability and larger active areas for analytes having lower sensitivity/permeability), but this approach may present significant manufacturing challenges and may not be applicable in all cases. Thus, the paucity of compositions available for forming mass transport limiting membranes may complicate the detection of certain analytes.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 shows a diagram of an illustrative sensing system that may incorporate an analyte sensor of the present disclosure.
FIGS. 2A-2C show cross-sectional diagrams of analyte sensors comprising a single active area.
FIGS. 3A-3C show cross-sectional diagrams of analyte sensors comprising two active areas.
FIG. 4 shows a cross-sectional diagram of an analyte sensor comprising two working electrodes, each having an active area present thereon.
FIG. 5 is a diagram showing a top view of a conventional carbon working electrode having an active area thereon.
FIG. 6A shows a cross-sectional diagram of a first configuration of a carbon working electrode suitable for use in the analyte sensors of the present disclosure. FIG. 6B shows a corresponding top view diagram.
FIG. 7 shows a diagram of an illustrative process whereby a carbon working electrode may be manufactured within a dielectric substrate.
FIG. 8A shows a cross-sectional diagram of a second configuration of a carbon working electrode suitable for use in the analyte sensors of the present disclosure. FIG. 8B shows a corresponding top view diagram.
FIG. 9A shows a cross-sectional diagram of a third configuration of a carbon working electrode suitable for use in the analyte sensors of the present disclosure. FIG. 9B shows a corresponding top view diagram.
FIGS. 10A and 10B show enzyme systems configured for detecting glucose.
FIGS. 11A-11C show enzyme systems configured for detecting ketones.
FIG. 12 shows an enzyme system configured for detecting creatinine.
FIG. 13 shows a plot of sensor response for several glucose sensors overcoated with a mass transport limiting membrane formed from HEMA:POMA at various ratios. FIG. 14 shows a corresponding plot of sensor response as a function of glucose concentration.
FIGS. 15A and 15B show plots of sensor response for several ketone sensors overcoated with a mass transport limiting membrane formed from HEMA: POMA at various ratios. FIGS. 16A and 16B show corresponding plots of sensor response as a function of ketone concentration.
FIG. 17 shows a plot of sensor response for several ketone sensors overcoated with a mass transport limiting membrane formed from a thiol-ene polymer at various ratios. FIG. 18 shows a corresponding plot of the sensor response as a function of ketone concentration.

### DETAILED DESCRIPTION

The present disclosure generally describes analyte sensors suitable for *in vivo* use and, more specifically, analyte sensors featuring one or more enhancements for promoting improved detection sensitivity and methods for production and use thereof. Such enhancements may include decreasing the amount of extraneous carbon upon the surface of a carbon working electrode and/or broadening the range of membrane chemistries available for deposition of mass transport limiting membranes. In particular, the range of available chemistries for mass transport limiting membranes may be expanded through *in situ* polymerization reaction, such as photopolymerization, to afford selective membrane deposition upon the active area(s) of a working electrode. The selectivity afforded by *in situ* photopolymerization may allow mass transport limiting membranes having differing compositions to be deposited upon selected areas of a working electrode, which may be particularly beneficial when assaying multiple analytes using a single analyte sensor capable of multi-analyte detection. Particular details and further advantages of each type of enhancement are described in further detail herein. Depending on particular needs, the analyte sensors of the present disclosure may be configured to detect one analyte or multiple analytes simultaneously or near simultaneously.

Analyte sensors employing enzyme-based detection are commonly used for assaying a single analyte, such as glucose, due to the frequent specificity of enzymes for a particular substrate or class of substrate. Analyte sensors employing both single enzymes and enzyme systems comprising multiple enzymes acting in concert may be used for this purpose. As used herein, the term "in concert" refers to a coupled enzymatic reaction, in which the product of a first enzymatic reaction becomes the substrate for a second enzymatic reaction, and the second enzymatic reaction or a subsequent enzymatic reaction serves as the basis for measuring the concentration of an analyte. Using an *in vivo* analyte sensor featuring an enzyme or enzyme system to promote detection may be particularly advantageous to avoid the frequent withdrawal of bodily fluid that otherwise may be required for analyte monitoring to take place.

Active areas suitable for assaying various analytes are now known, but there are sometimes difficulties associated with detection of certain analytes or combinations of analytes. In the case of low-abundance analytes, for example, the sensor sensitivity may be insufficient in some cases. A carbon working electrode may provide a relatively high background signal that may complicate the accurate detection of some low-abundance analytes. When assaying multiple analytes, significantly different membrane permeability values may also be problematic. The present disclosure provides analyte sensor enhancements that, either alone or in combination, may improve detection sensitivity for both single analytes and multiple analytes in combination with one another, as explained in further detail hereinbelow. Namely, the present disclosure provides analyte sensors having carbon working electrodes that may afford decreased background signal and an expanded range of suitable membrane chemistry that may be selectively deposited upon an analyte sensor. Although certain aspects of the present disclosure are directed to enhancement of carbon working electrodes, it is to be appreciated that other types of electrodes may be similarly enhanced according to the disclosure herein. Electrode types that may be enhanced through use of the disclosure herein also include gold, platinum, PEDOT, and the like.

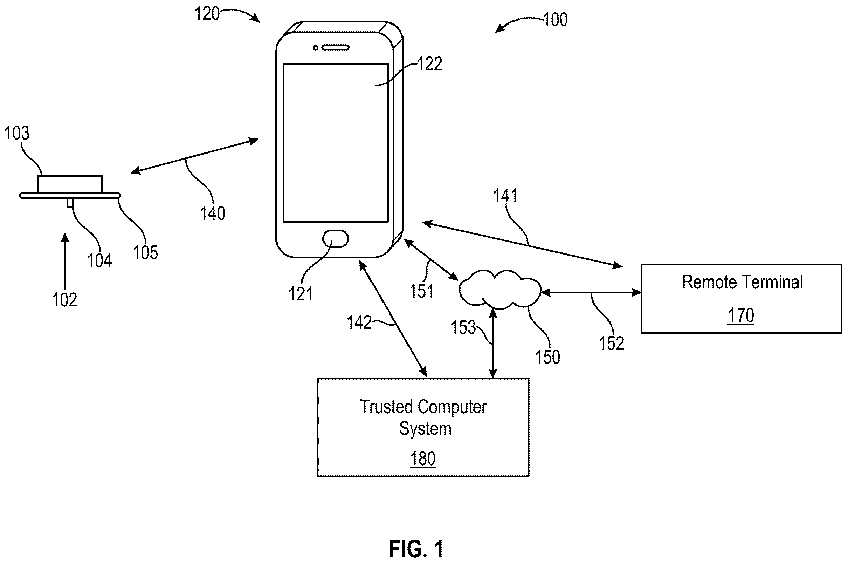

Before describing the analyte sensors of the present disclosure and their enhancements in further detail, a brief overview of suitable *in vivo* analyte sensor configurations and sensor systems employing the analyte sensors will be provided first so that the embodiments of the present disclosure may be better understood. FIG. 1 shows a diagram of an illustrative sensing system that may incorporate an analyte sensor of the present disclosure. As shown, sensing system 100 includes sensor control device 102 and reader device 120 that are configured to communicate with one another over local communication path or link 140, which may be wired or wireless, uni- or bi-directional, and encrypted or non-encrypted. Reader device 120 may constitute an output medium for viewing analyte concentrations and alerts or notifications determined by sensor 104 or a processor associated therewith, as well as allowing for one or more user inputs, according to some embodiments. Reader device 120 may be a multi-purpose smartphone or a dedicated electronic reader instrument. While only one reader device 120 is shown, multiple reader devices 120 may be present in certain instances. Reader device 120 may also be in communication with remote terminal 170 and/or trusted computer system 180 via communication path(s)/link(s) 141 and/or 142, respectively, which also may be wired or wireless, uni- or bi-directional, and encrypted or non-encrypted. Reader device 120 may also or alternately be in communication with network 150 (*e.g.,* a mobile telephone network, the internet, or a cloud server) via communication path/link 151. Network 150 may be further communicatively coupled to remote terminal 170 via communication path/link 152 and/or trusted computer system 180 via communication path/link 153. Alternately, sensor 104 may communicate directly with remote terminal 170 and/or trusted computer system 180 without an intervening reader device 120 being present. For example, sensor 104 may communicate with remote terminal 170 and/or trusted computer system 180 through a direct communication link to network 150, according to some embodiments, as described in U.S. Patent Application Publication 2011/0213225 and incorporated herein by reference in its entirety. Any suitable electronic communication protocol may be used for each of the communication paths or links, such as near field communication (NFC), radio frequency identification (RFID), BLUETOOTH^{®} or BLUETOOTH^{®} Low Energy protocols, WiFi, or the like. Remote terminal 170 and/or trusted computer system 180 may be accessible, according to some embodiments, by individuals other than a primary user who have an interest in the user's analyte levels. Reader device 120 may comprise display 122 and optional input component 121. Display 122 may comprise a touch-screen interface, according to some embodiments.

Sensor control device 102 includes sensor housing 103, which may house circuitry and a power source for operating sensor 104. Optionally, the power source and/or active circuitry may be omitted. A processor (not shown) may be communicatively coupled to sensor 104, with the processor being physically located within sensor housing 103 or reader device 120. Sensor 104 protrudes from the underside of sensor housing 103 and extends through adhesive layer 105, which is adapted for adhering sensor housing 103 to a tissue surface, such as skin, according to some embodiments.

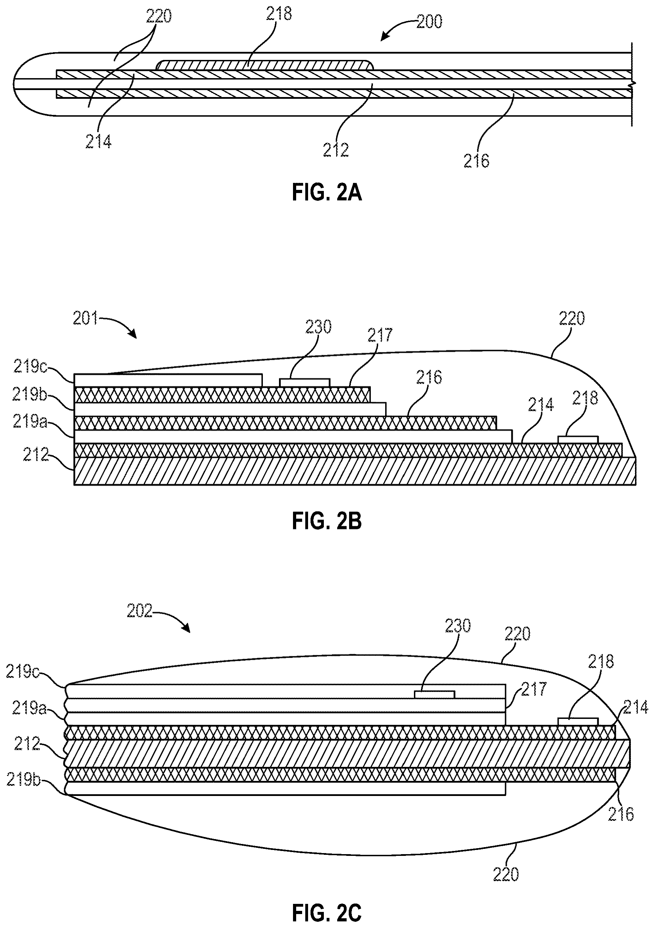

Sensor 104 is adapted to be at least partially inserted into a tissue of interest, such as within the dermal or subcutaneous layer of the skin. Alternately, sensor 104 may be adapted to penetrate the epidermis. Still further alternately, sensor 104 may be disposed superficially and not penetrate a tissue, such as when assaying one or more analytes in perspiration upon the skin. Sensor 104 may comprise a sensor tail of sufficient length for insertion to a desired depth in a given tissue. The sensor tail may comprise at least one working electrode and an active area comprising an enzyme or enzyme system configured for assaying one or more analytes of interest. Suitable enzymes and enzyme systems are discussed in further detail herein. A counter electrode may be present in combination with the at least one working electrode, optionally in further combination with a reference electrode. Particular electrode configurations upon the sensor tail are described in more detail below in reference to FIGS. 2A-4. One or more enzymes in the active area(s) may be covalently bonded to a polymer comprising the active area(s), according to various embodiments. Alternately, enzymes may be non-covalently associated within the active area(s), such as through encapsulation or physical entrainment. The one or more analytes may be monitored in any biological fluid of interest such as dermal fluid, interstitial fluid, plasma, blood, lymph, synovial fluid, cerebrospinal fluid, saliva, bronchoalveolar lavage, amniotic fluid, or the like. In particular embodiments, analyte sensors of the present disclosure may be adapted for assaying dermal fluid or interstitial fluid to determine analyte concentrations *in vivo.*

One or more mass transport limiting membranes may overcoat the active area(s). When different types of active areas are present, the mass transport limiting membrane may be compositionally the same or compositionally different at each type of active area. Sensor architectures are available for incorporating, via dip coating techniques, different mass transport limiting membranes upon each type of active area, when needed, to facilitate detection of multiple analytes. Different membrane thicknesses or membrane architectures may also be employed to facilitate detection of multiple analytes. As described further herein, mass transport limiting membranes having compositional variation at one or more of the active areas may also be prepared through *in situ* polymerization, such as *in situ* photopolymerization. Living polymerization techniques may also be employed to form a mass transport limiting membrane upon an active area. If a single mass transport limiting membrane provides satisfactory permeability for both analytes detectable by a multi-analyte detector, a simpler sensor architecture may be used. *In situ* photopolymerization may also expand the range of suitable membrane chemistries beyond those that are only suitable for deposition by dip-coating techniques. Better quality membranes may also be formed in some cases, as compared to dip coating techniques.

Referring again to FIG. 1, sensor 104 may automatically forward data to reader device 120. For example, analyte concentration data may be communicated automatically and periodically, such as at a certain frequency as data is obtained or after a certain time period has passed, with the data being stored in a memory until transmittal (*e*.*g*., every minute, five minutes, or other predetermined time period). Data associated with different analytes may be forwarded at the same frequency or different frequencies and/or using the same or different communication protocols. In other embodiments, sensor 104 may communicate with reader device 120 in a non-automatic manner and not according to a set schedule. For example, data may be communicated from sensor 104 using RFID technology when the sensor electronics are brought into communication range of reader device 120. Until communicated to reader device 120, data may remain stored in a memory of sensor 104. Thus, a user does not have to maintain close proximity to reader device 120 at all times, and can instead upload data at a convenient time. In yet other embodiments, a combination of automatic and non-automatic data transfer may be implemented. For example, data transfer may continue on an automatic basis until reader device 120 is no longer in communication range of sensor 104.

An introducer may be present transiently to promote introduction of sensor 104 into a tissue. In illustrative embodiments, the introducer may comprise a needle or similar sharp, or a combination thereof. It is to be recognized that other types of introducers, such as sheaths or blades, may be present in alternative embodiments. More specifically, the needle or other introducer may transiently reside in proximity to sensor 104 prior to tissue insertion and then be withdrawn afterward. While present, the needle or other introducer may facilitate insertion of sensor 104 into a tissue by opening an access pathway for sensor 104 to follow. For example, the needle may facilitate penetration of the epidermis as an access pathway to the dermis to allow implantation of sensor 104 to take place, according to one or more embodiments. After opening the access pathway, the needle or other introducer may be withdrawn so that it does not represent a sharps hazard. In illustrative embodiments, suitable needles may be solid or hollow, beveled or non-beveled, and/or circular or non-circular in cross-section. In more particular embodiments, suitable needles may be comparable in cross-sectional diameter and/or tip design to an acupuncture needle, which may have a cross-sectional diameter of about 250 microns. It is to be recognized, however, that suitable needles may have a larger or smaller cross-sectional diameter if needed for particular applications. For example, needles having a cross-sectional diameter ranging from about 300 microns to about 400 microns may be used.

In some embodiments, a tip of the needle (while present) may be angled over the terminus of sensor 104, such that the needle penetrates a tissue first and opens an access pathway for sensor 104. In other illustrative embodiments, sensor 104 may reside within a lumen or groove of the needle, with the needle similarly opening an access pathway for sensor 104. In either case, the needle may be subsequently withdrawn after facilitating sensor insertion.

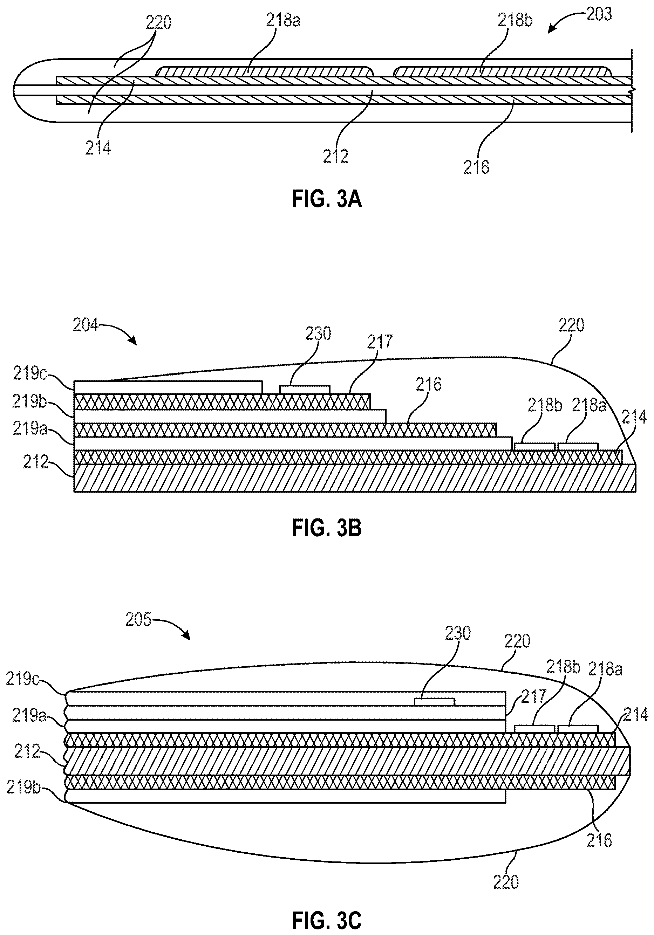

Sensor configurations featuring a single active area that is configured for detection of a corresponding single analyte may employ two-electrode or three-electrode detection motifs, as described further herein in reference to FIGS. 2A-2C. Sensor configurations featuring two different active areas for detection of separate analytes, either upon separate working electrodes or upon the same working electrode, are described separately thereafter in reference to FIGS. 3A-4. Sensor configurations having multiple working electrodes may be particularly advantageous for incorporating two different active areas within the same sensor tail, since the signal contribution from each active area may be determined more readily through separate interrogation of each working electrode. Each active area may be overcoated with a mass transport limiting membrane, which may be introduced through dip coating or produced through *in situ* photopolymerization in accordance with the further disclosure below.

When a single working electrode is present in an analyte sensor, three-electrode sensor configurations may comprise a working electrode, a counter electrode, and a reference electrode. Related two-electrode sensor configurations may comprise a working electrode and a second electrode, in which the second electrode may function as both a counter electrode and a reference electrode (*i.e.,* a counter/reference electrode). The various electrodes may be at least partially stacked (layered) upon one another and/or laterally spaced apart from one another upon the sensor tail. In any of the sensor configurations disclosed herein, the various electrodes may be electrically isolated from one another by a dielectric material or similar insulator.

Analyte sensors featuring multiple working electrodes may similarly comprise at least one additional electrode. When one additional electrode is present, the one additional electrode may function as a counter/reference electrode for each of the multiple working electrodes. When two additional electrodes are present, one of the additional electrodes may function as a counter electrode for each of the multiple working electrodes and the other of the additional electrodes may function as a reference electrode for each of the multiple working electrodes.

FIG. 2A shows a diagram of an illustrative two-electrode analyte sensor configuration, which is compatible for use in the disclosure herein. As shown, analyte sensor 200 comprises substrate 212 disposed between working electrode 214 and counter/reference electrode 216. Alternately, working electrode 214 and counter/reference electrode 216 may be located upon the same side of substrate 212 with a dielectric material interposed in between (configuration not shown). Active area 218 is disposed as at least one layer upon at least a portion of working electrode 214. Active area 218 may comprise multiple spots or a single spot configured for detection of an analyte, as discussed further herein.

Referring still to FIG. 2A, membrane 220 overcoats at least active area 218 and may optionally overcoat some or all of working electrode 214 and/or counter/reference electrode 216, or the entirety of analyte sensor 200, according to some embodiments. One or both faces of analyte sensor 200 may be overcoated with membrane 220. Membrane 220 may comprise one or more polymeric membrane materials having capabilities of limiting analyte flux to active area 218 (*i.e.,* membrane 220 is a mass transport limiting membrane having some permeability for the analyte of interest). The composition and thickness of membrane 220 may vary to promote a desired analyte flux to active area 218, thereby providing a desired signal intensity and stability. Analyte sensor 200 may be operable for assaying an analyte by any of coulometric, amperometric, voltammetric, or potentiometric electrochemical detection techniques.

FIGS. 2B and 2C show diagrams of illustrative three-electrode analyte sensor configurations, which are also compatible for use in the disclosure herein. Three-electrode analyte sensor configurations may be similar to that shown for analyte sensor 200 in FIG. 2A, except for the inclusion of additional electrode 217 in analyte sensors 201 and 202 (FIGS. 2B and 2C). With additional electrode 217, counter/reference electrode 216 may then function as either a counter electrode or a reference electrode, and additional electrode 217 fulfills the other electrode function not otherwise accounted for. Working electrode 214 continues to fulfill its original function. Additional electrode 217 may be disposed upon either working electrode 214 or electrode 216, with a separating layer of dielectric material in between. For example, as depicted in FIG. 2B, dielectric layers 219a, 219b and 219c separate electrodes 214, 216 and 217 from one another and provide electrical isolation. Alternately, at least one of electrodes 214, 216 and 217 may be located upon opposite faces of substrate 212, as shown in FIG. 2C. Thus, in some embodiments, electrode 214 (working electrode) and electrode 216 (counter electrode) may be located upon opposite faces of substrate 212, with electrode 217 (reference electrode) being located upon one of electrodes 214 or 216 and spaced apart therefrom with a dielectric material. Reference material layer 230 (*e*.*g*., Ag/AgCl) may be present upon electrode 217, with the location of reference material layer 230 not being limited to that depicted in FIGS. 2B and 2C. As with sensor 200 shown in FIG. 2A, active area 218 in analyte sensors 201 and 202 may comprise multiple spots or a single spot. Additionally, analyte sensors 201 and 202 may likewise be operable for assaying an analyte by any of coulometric, amperometric, voltammetric, or potentiometric electrochemical detection techniques.

Like analyte sensor 200, membrane 220 may also overcoat active area 218, as well as other sensor components, in analyte sensors 201 and 202, thereby serving as a mass transport limiting membrane. Additional electrode 217 may be overcoated with membrane 220 in some embodiments. Membrane 220 may again be produced through dip coating or *in situ* photopolymerization and vary compositionally at different locations. Although FIGS. 2B and 2C have depicted all of electrodes 214, 216 and 217 as being overcoated with membrane 220, it is to be recognized that only working electrode 214 or active area 218 may be overcoated in some embodiments. Moreover, the thickness of membrane 220 at each of electrodes 214, 216 and 217 may be the same or different. As in two-electrode analyte sensor configurations (FIG. 2A), one or both faces of analyte sensors 201 and 202 may be overcoated with membrane 220 in the sensor configurations of FIGS. 2B and 2C, or the entirety of analyte sensors 201 and 202 may be overcoated. Accordingly, the three-electrode sensor configurations shown in FIGS. 2B and 2C should be understood as being non-limiting of the embodiments disclosed herein, with alternative electrode and/or layer configurations remaining within the scope of the present disclosure.

FIG. 3A shows an illustrative configuration for sensor 203 having a single working electrode with two different active areas disposed thereon. FIG. 3A is similar to FIG. 2A, except for the presence of two active areas upon working electrode 214: first active area 218a and second active area 218b, which are responsive to different analytes and are laterally spaced apart from one another upon the surface of working electrode 214. Active areas 218a and 218b may comprise multiple spots or a single spot configured for detection of each analyte. The composition of membrane 220 may vary or be compositionally the same at active areas 218a and 218b. First active area 218a and second active area 218b may be configured to detect their corresponding analytes at working electrode potentials that differ from one another, as discussed further below.

FIGS. 3B and 3C show cross-sectional diagrams of illustrative three-electrode sensor configurations for sensors 204 and 205, respectively, each featuring a single working electrode having first active area 218a and second active area 218b disposed thereon. FIGS. 3B and 3C are otherwise similar to FIGS. 2B and 2C and may be better understood by reference thereto. As with FIG. 3A, the composition of membrane 220 may vary or be compositionally the same at active areas 218a and 218b.

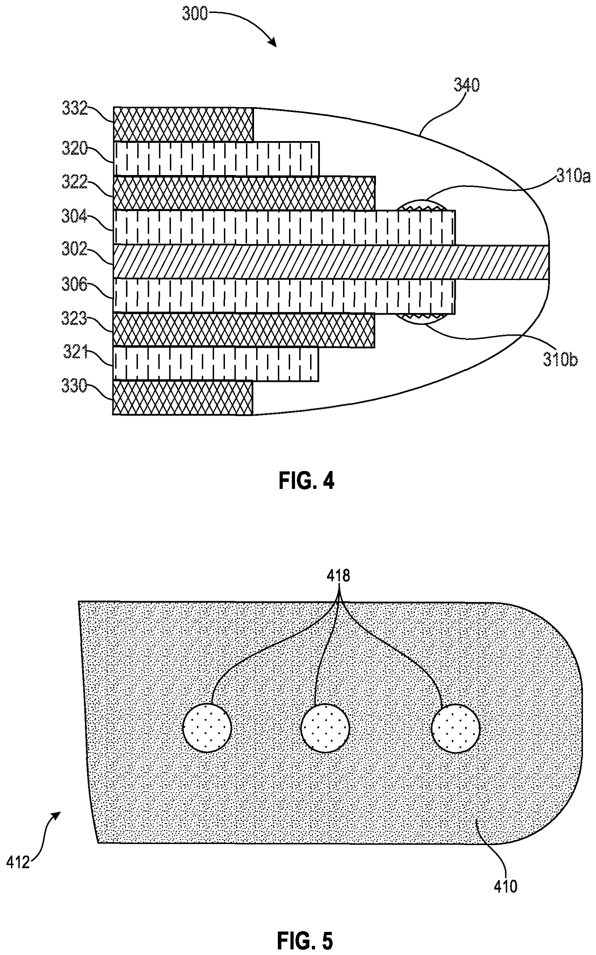

Illustrative sensor configurations having multiple working electrodes, specifically two working electrodes, are described in further detail in reference to FIG. 4. Although the following description is primarily directed to sensor configurations having two working electrodes, it is to be appreciated that more than two working electrodes may be incorporated through extension of the disclosure herein. Additional working electrodes may be used to impart additional sensing capabilities to the analyte sensors beyond just a first analyte and a second analyte. That is, analyte sensors containing more than two working electrodes may be suitable for detecting a commensurate number of additional analytes.

FIG. 4 shows a cross-sectional diagram of an illustrative analyte sensor configuration having two working electrodes, a reference electrode and a counter electrode, which is compatible for use in the disclosure herein. As shown, analyte sensor 300 includes working electrodes 304 and 306 disposed upon opposite faces of substrate 302. First active area 310a is disposed upon the surface of working electrode 304, and second active area 310b is disposed upon the surface of working electrode 306. Counter electrode 320 is electrically isolated from working electrode 304 by dielectric layer 322, and reference electrode 321 is electrically isolated from working electrode 306 by dielectric layer 323. Outer dielectric layers 330 and 332 are positioned upon reference electrode 321 and counter electrode 320, respectively. Membrane 340 may overcoat at least active areas 310a and 310b, according to various embodiments, with other components of analyte sensor 300 or the entirety of analyte sensor 300 optionally being overcoated with membrane 340 as well. Again, membrane 340 may vary compositionally at active areas 310a and 310b, if needed, in order to afford suitable permeability values for differentially regulating the analyte flux at each location.

Alternative sensor configurations having multiple working electrodes and differing from the configuration shown in FIG. 4 may feature a counter/reference electrode instead of separate counter and reference electrodes 320,321, and/or feature layer and/or membrane arrangements varying from those expressly depicted. For example, the positioning of counter electrode 320 and reference electrode 321 may be reversed from that depicted in FIG.4. In addition, working electrodes 304 and 306 need not necessarily reside upon opposing faces of substrate 302 in the manner shown in FIG. 4.

A carbon working electrode may suitably comprise the working electrode(s) in any of the analyte sensors disclosed herein. While carbon working electrodes are very commonly employed in electrochemical detection, use thereof in electrochemical sensing is not without difficulties. In particular, current related to an analyte of interest only results when an active area interacts with an analyte and transfers electrons to the portion of the carbon working electrode adjacent to the active area. Fluid containing an analyte of interest also interacts with a carbon surface of the carbon working electrode not overcoated with an active area and does not contribute to the analyte signal, since there is no enzyme or enzyme system present at these locations to facilitate electron transfer from the analyte to the working electrode. Interferents may, however, undergo oxidation at the working electrode portions lacking an active area and contribute background to the overall signal. Ascorbic acid is one example of an interferent commonly present in biological fluids that may generate a background signal at a carbon working electrode. Thus, carbon working electrodes with an extraneous carbon area upon the electrode surface do not meaningfully contribute to the analyte signal and may lead to excessive background in some cases. Other electrodes having an excessive surface area not directly detecting an analyte of interest may experience similar problems and may be enhanced through modification of the disclosure herein.

FIG. 5 is a diagram showing a top view of conventional carbon working electrode 412 having active area 418 disposed as multiple spots thereon. Only portions of carbon working electrode 412 beneath active area 418 contribute signal associated with an analyte of interest when the analyte interacts with active area 418. Extraneous carbon area 410 is not directly overlaid with active area 418 and does not contribute signal associated with the analyte but may generate a background signal associated with one or more interferents.

The present disclosure demonstrates how extraneous carbon area may be decreased in a carbon working electrode while still retaining functionality for producing a signal associated with an analyte of interest. In particular, the present disclosure provides analyte sensors comprising modified carbon working electrodes formed at least partially within a dielectric substrate, such as a polymer block, wherein the dielectric substrate replaces a majority or all of the extraneous carbon area upon the electrode surface not overlaid with an active area. More specifically, the carbon working electrodes described herein may be obtained through filling of one or more apertures with a carbon conductor, as described in greater detail hereinbelow. Alternative electrodes comprising metal or PEDOT may be formed similarly within a dielectric substrate in accordance with the disclosure herein.

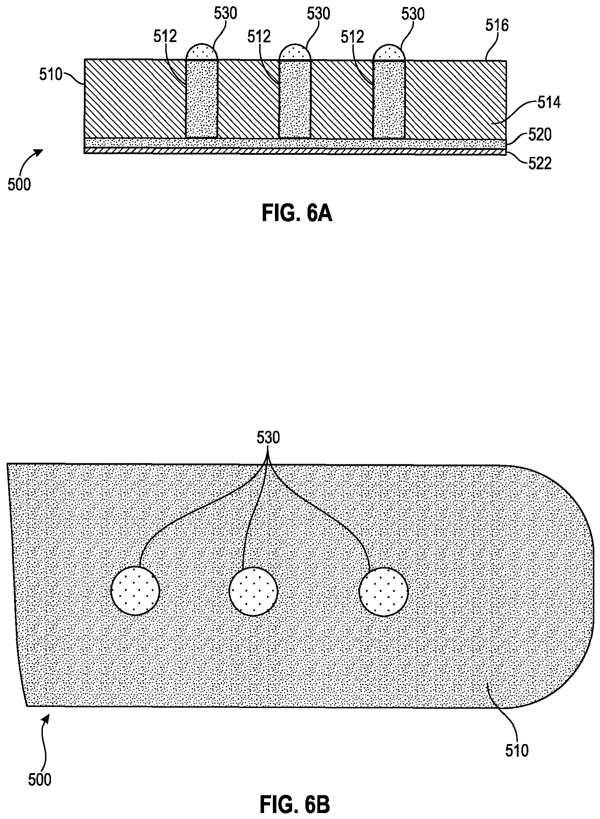

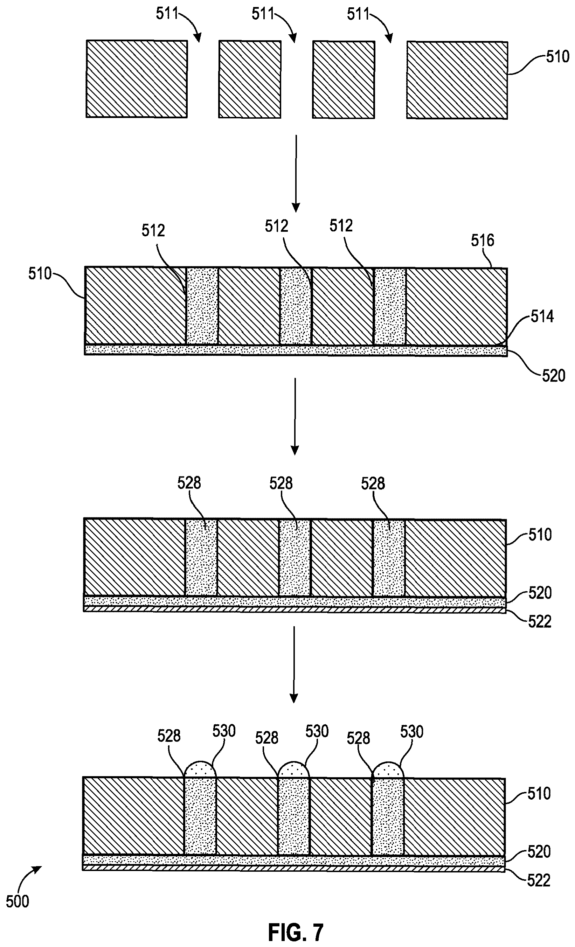

FIG. 6A shows a cross-sectional diagram of a first configuration of a carbon working electrode suitable for use in the analyte sensors of the present disclosure. As shown in FIG. 6A, carbon working electrode 500 contains dielectric substrate 510 having carbon conductor pillars 512 extending therethrough between first face 514 and second face 516. Carbon conductor pillars 512 may be formed by filling a via or similar aperture with a carbon conductor ink, followed by curing, as described in greater detail hereinafter in reference to FIG. 7. Carbon conductor coating 520 is disposed upon first face 514 and directly contacts each of carbon conductor pillars 512, thereby establishing electrical communication among each of carbon conductor pillars 512. Dielectric coating 522 is disposed upon carbon conductor coating 520. Thus, the only available carbon surface for transferring electrons is located upon second face 516 where carbon conductor pillars 512 break through dielectric substrate 510. Active areas 530 are disposed upon second face 516 in direct contact with carbon conductor pillars 512.

Alternatives to carbon working electrodes may be formed by depositing a similar conductive ink within an aperture extending through dielectric substrate 510. Metal-containing inks, for example, may be deposited and cured similarly to afford metallic conductor pillars. A metallic conductor coating may interconnect the metallic conductor pillars and may be formed similarly from a metal-containing ink. Accordingly, any electrode structure herein described in reference to a carbon working electrode may be formed similarly with alternative conductive materials.

FIG. 6B shows a corresponding top view of the carbon working electrode of FIG. 6A. As shown, active areas 530 overlay the exposed surface of carbon conductor pillars 512 (not visible in depicted view), such that little to no extraneous carbon area remains available for generating an electrochemical signal upon interacting with an interferent. Although FIGS. 6A and 6B have shown complete overlay of active areas 530 upon carbon conductor pillars 512, it is to be appreciated that active areas 530 may also be slightly offset from carbon conductor pillars 512 and/or not completely overlay carbon conductor pillars 512 as well, thereby leaving a small amount of extraneous carbon area that may be exposed to an interferent. Nevertheless, it can be appreciated that the amount of extraneous carbon present in carbon working electrode 500 is much less than that in a conventional carbon working electrode having a face formed substantially of a carbon conductor. It is also to be appreciated that the number and spacing of carbon conductor pillars 512 and active areas 530 are illustrative and non-limiting. In illustrative embodiments, the number of apertures (vias) may range from about 1 to about 20, or about 3 to about 15, or about 2 to about 10, and the diameter of the apertures may range from about 25 µm to about 200 µm. Non-circular apertures may be present as well. A corresponding number of active areas may be present upon the carbon conductor pillars, or the number of active areas may be less than the number of carbon conductor pillars. If less than all of the carbon conductor pillars are overcoated with an active area, some extraneous carbon will remain present for potential exposure to an interferent.

FIG. 7 shows a diagram of an illustrative process whereby a carbon working electrode may be manufactured within a dielectric substrate. First, dielectric substrate 510 is provided with a plurality of vias 511 extending therethrough. There are numerous ways to introduce vias 511 into dielectric substrate 510, such as drilling, laser drilling, or like techniques familiar to one having ordinary skill in the art. A carbon conductor ink is then deposited by screen printing or a similar deposition technique to form carbon conductor coating 520 upon first face 514. Vias 511 may become at least partially filled during this process as well. If needed, completion of the filling of vias 511 with the carbon conductor ink to afford carbon conductor pillars 512 after ink drying may then take place from second face 516. Suitable carbon conductor inks will be familiar to one having ordinary skill in the art.

Dielectric coating 522 is then deposited upon carbon conductor coating 520, leaving exposed carbon conductor pillar surfaces 528 upon second face 516 of dielectric substrate 510. Finally, active areas 530 are deposited upon exposed carbon conductor pillar surfaces 528 to afford carbon working electrode 500 having sensing capabilities for an analyte of interest. Although FIG. 7 has been described with respect to via filling, it is to be appreciated that other types of apertures may be filled with a carbon conductor ink to form similar types of carbon conductor pillars therein.

Accordingly, methods for forming an analyte sensor of the present disclosure may comprise: providing a dielectric substrate having one or more apertures extending therethrough between a first face and a second face; filling the one or more apertures with a carbon conductor to form carbon conductor pillars therein and depositing a carbon conductor coating on the first face of the dielectric substrate, the carbon conductor coating being in direct contact with each carbon conductor pillar; depositing a dielectric coating upon the carbon conductor coating; forming one or more active areas upon the second face of the dielectric substrate in electrical communication with the carbon conductor pillars within the one or more apertures, the one or more active areas being responsive to an analyte; and depositing a mass transport limiting membrane upon at least the one or more active areas.

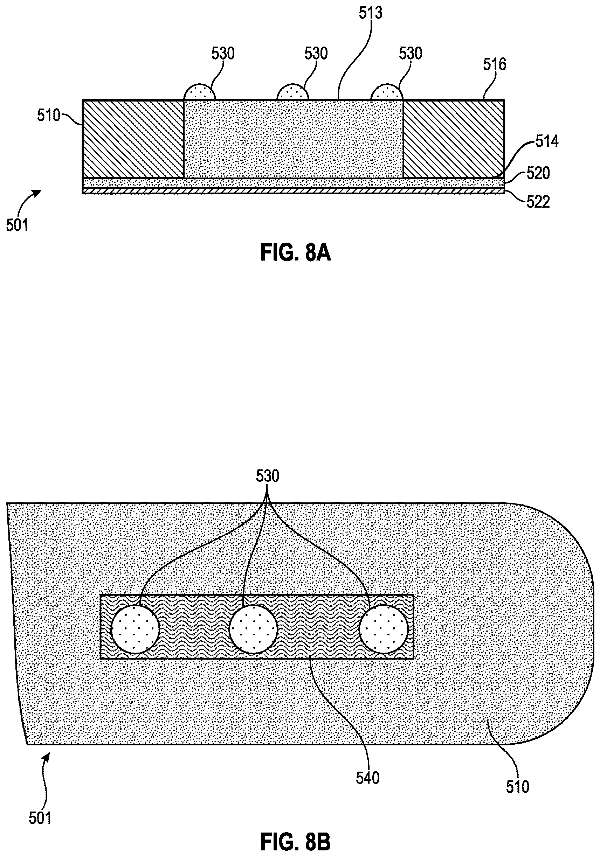

FIG. 8A shows a cross-sectional diagram of a second configuration of a carbon working electrode suitable for use in the analyte sensors of the present disclosure. Carbon working electrode 501 differs from carbon working electrode 500 depicted in FIGS. 6A and 6B in that monolithic carbon conductor pillar 513 extends through dielectric substrate 510 between first face 514 and second face 516, thereby replacing multiple, smaller carbon conductor pillars 512. Monolithic carbon conductor pillar 513 may be formed by filling a slot or similar aperture with a carbon conductor ink, followed by ink drying, in a like manner to that described above in reference to FIG. 7. Each of active areas 530 are disposed upon second face 516 in direct contact with monolithic carbon conductor pillar 513.

FIG. 8B shows a corresponding top view of the carbon working electrode of FIG. 8A. As shown, active areas 530 overlay exposed surface 540 of monolithic carbon conductor pillar 513. Although exposed surface 540 is not completely covered by active areas 530 in carbon working electrode 501, the amount of extraneous carbon is much less than that present in a conventional carbon working electrode having a surface formed substantially of a carbon conductor. It is again to be appreciated that the number and spacing of active areas 530 are illustrative and non-limiting. In illustrative embodiments, the slot may range from about 25 µm to about 200 µm wide and about 100 µm to about 2000 µm long.

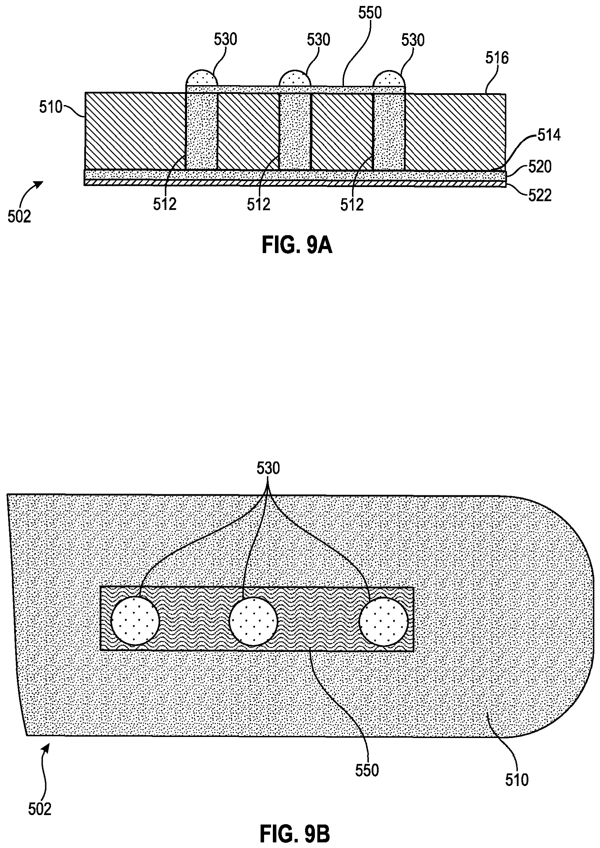

FIG. 9A shows a cross-sectional diagram of a third configuration of a carbon working electrode suitable for use in the analyte sensors of the present disclosure. Carbon working electrode 502 differs from carbon working electrode 500 depicted in FIGS. 6A and 6B in that carbon conductor pillars 512 are interconnected by carbon conductor strip 550 located upon second face 516 of dielectric substrate 510. Active areas 530 are disposed directly upon carbon strip 550, rather than upon individual carbon conductor pillars 512 in carbon working electrode 501. Use of carbon conductor strip 550 ensures that active areas 530 are all electrically connected to carbon conductor coating 520. For example, if there is incomplete via fill when forming one or more of carbon conductor pillars 512, due to manufacturing issues, electrical conductivity of a given active area 530 to carbon conductor coating 520 may still be maintained by carbon conductor strip 550. Moreover, in electrode configurations employing carbon conductor strip 550, active areas 530 need not directly overlay each carbon conductor pillar 512.

FIG. 9B shows a corresponding top view of the carbon working electrode of FIG. 9A. The top view of carbon working electrode 502 is similar to that of carbon working electrode 501, as shown in FIG. 8B, except that carbon conductor strip 550 overlays individual carbon conductor pillars 512, rather than monolithic carbon conductor pillar 513 providing the entirety of the extraneous carbon upon second surface 516 and affording a deposition surface for active areas 530. Like carbon working electrode 501, carbon conductor strip 550 is not completely covered by active areas 530 in carbon working electrode 502, but the amount of extraneous carbon is much less than that present in a conventional carbon working electrode having a surface formed substantially of a carbon conductor. It is again to be appreciated that the number and spacing of carbon conductor pillars 512 and active areas 530 are illustrative and non-limiting. In illustrative embodiments, the number of apertures (vias) may range from about 1 to about 20, or about 3 to about 15, or about 2 to about 10, and the diameter of the apertures may range from about 25 µm to about 200 µm. The carbon conductive strip interconnecting the carbon conductor pillars may range from about 25 µm to about 200 µm wide and about 100 µm to about 2000 µm long.

Accordingly, the present disclosure provides analyte sensors comprising a carbon working electrode having a dielectric substrate, one or more apertures extending through the dielectric substrate in which the one or more apertures are filled with a carbon conductor pillar extending between a first face and a second face of the dielectric substrate, and a carbon conductor coating disposed upon the first face of the dielectric substrate in direct contact with each carbon conductor pillar. One or more active areas are located upon the second face of the dielectric substrate that are in electrical communication with each of the carbon conductor pillars, in which the one or more active areas are responsive to an analyte. A mass transport limiting membrane may overcoat at least the one or more active areas.

The one or more apertures extending through the dielectric substrate may be in the form of multiple vias extending through the dielectric substrate or a slot extending through the dielectric substrate. The carbon conductor pillar may be disposed within the vias or the slot and completely fill the vias or the slot. The active areas may be disposed directly upon the carbon conductor pillar(s), located within either type of aperture, upon the second face of the dielectric substrate.

Optionally, multiple vias extending through the dielectric substrate may be electrically interconnected through a carbon conductor strip located upon the second face of the dielectric substrate and overcoating the multiple vias. The active areas may be disposed directly upon the carbon conductor strip and need not necessarily overlay a corresponding via. Although a carbon conductor strip introduces some extraneous carbon to the electrode surface, improved manufacturing reliability may outweigh enhancement of sensor performance in some cases. A layer thickness for the carbon conductor strip may range from about 1 micron to about 20 microns, for example.

Active areas within any of the analyte sensors disclosed herein may comprise one or more analyte-responsive enzymes, either acting alone or in concert within an enzyme system. One or more enzymes may be covalently bonded to a polymer comprising the active area, as can one or more electron transfer agents located within the active area.

Examples of suitable polymers within each active area may include poly(4-vinylpyridine) and poly(N-vinylimidazole) or a copolymer thereof, for example, in which quaternized pyridine and imidazole groups serve as a point of attachment for an electron transfer agent or enzyme(s). Other suitable polymers that may be present in the active area(s) include, but are not limited to, those described in U.S. Patent 6,605,200, incorporated herein by reference in its entirety, such as poly(acrylic acid), styrene/maleic anhydride copolymer, methylvinylether/maleic anhydride copolymer (GANTREZ polymer), poly(vinylbenzylchloride), poly(allylamine), polylysine, poly(4-vinylpyridine) quaternized with carboxypentyl groups, and poly(sodium 4-styrene sulfonate).

Enzymes covalently bound to the polymer in the active areas that are capable of promoting analyte detection are not believed to be particularly limited. Suitable enzymes may include those capable of detecting glucose, lactate, ketones, creatinine, or the like. Any of these analytes may be detected in combination with one another in analyte sensors capable of detecting multiple analytes. Suitable enzymes and enzyme systems for detecting these analytes are described hereinafter.

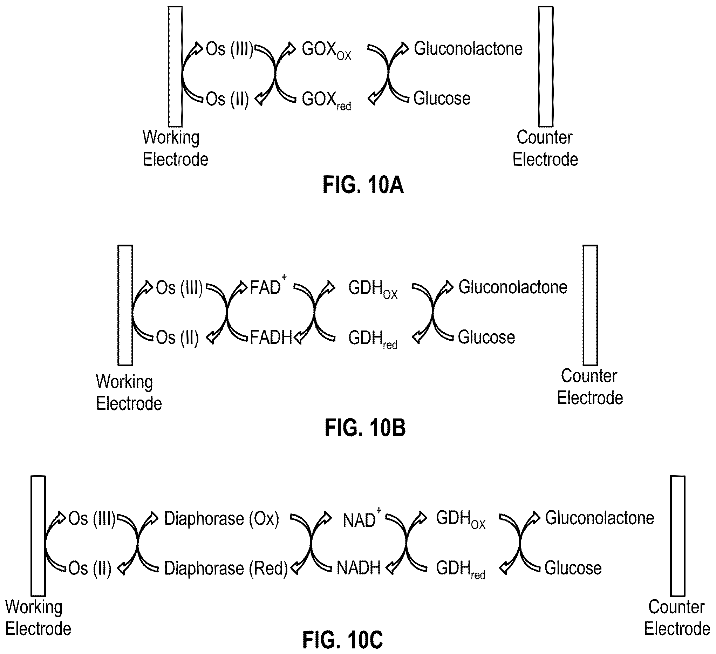

In some embodiments, the analyte sensors may comprise a glucose-responsive active area comprising a glucose-responsive enzyme disposed upon the sensor tail. Suitable glucose-responsive enzymes may include, for example, glucose oxidase or a glucose dehydrogenase (*e*.*g*., pyrroloquinoline quinone (PQQ) or a cofactor-dependent glucose dehydrogenase, such as flavine adenine dinucleotide (FAD)-dependent glucose dehydrogenase or nicotinamide adenine dinucleotide (NAD)-dependent glucose dehydrogenase). Glucose oxidase and glucose dehydrogenase are differentiated by their ability to utilize oxygen as an electron acceptor when oxidizing glucose; glucose oxidase may utilize oxygen as an electron acceptor, whereas glucose dehydrogenases transfer electrons to natural or artificial electron acceptors, such as an enzyme cofactor. Illustrative enzyme-based detection schemes for analyzing glucose are further shown in FIGS. 10A-10C, which utilize glucose oxidase or glucose dehydrogenase to promote detection. Both glucose oxidase and glucose dehydrogenase may be covalently bonded to a polymer comprising the glucose-responsive active area and exchange electrons with an electron transfer agent (*e*.*g*., an osmium (Os) complex or similar transition metal complex), which may also be covalently bonded to the polymer. Suitable electron transfer agents are described in further detail below. Glucose oxidase may directly exchange electrons with the electron transfer agent (FIG. 10A), whereas glucose dehydrogenase may utilize a cofactor to promote electron exchange with the electron transfer agent (FIGS. 10B and 10C). FAD cofactor may directly exchange electrons with the electron transfer agent, as shown in FIG. 10B. NAD cofactor, in contrast, may utilize diaphorase to facilitate electron transfer from the cofactor to the electron transfer agent, as shown in FIG. 10C. Further details concerning glucose-responsive active areas incorporating glucose oxidase or glucose dehydrogenase, as well as glucose detection therewith, may be found in commonly owned U.S. Patent 8,268,143, for example.

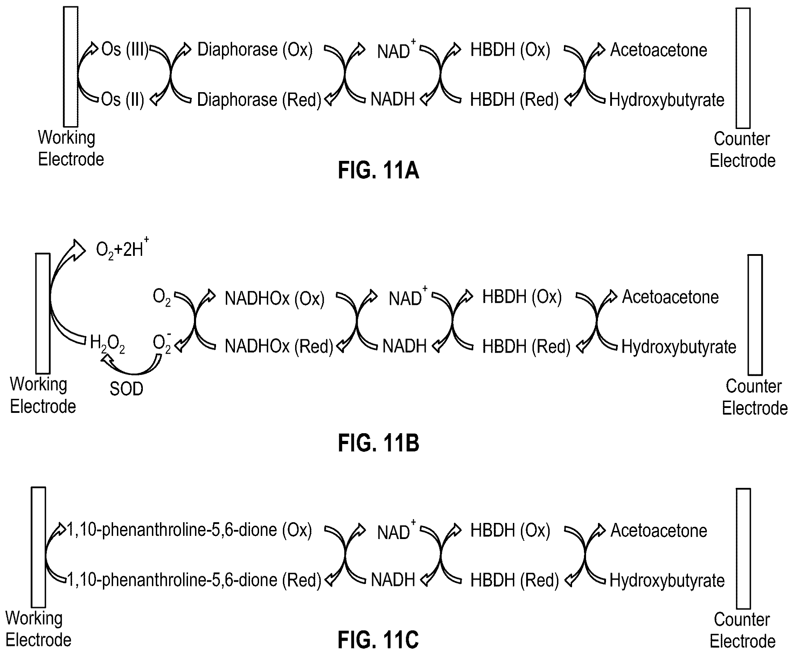

FIGS. 11A-11C show enzyme systems configured for detecting ketones. Additional details concerning enzyme systems responsive to ketones may be found in commonly owned U.S. Patent Application 16/774,835 entitled "Analyte Sensors and Sensing Methods Featuring Dual Detection of Glucose and Ketones," filed on January 28, 2020, and published as U.S. Patent Application Publication ________. In the enzyme system shown in FIG. 11A, β-hydroxybutyrate serves as a surrogate for ketones formed *in vivo,* which undergoes a reaction with an enzyme system comprising β-hydroxybutyrate dehydrogenase (HBDH) and diaphorase to facilitate ketones detection within a ketones-responsive active area disposed upon the surface of at least one working electrode, as described further herein. Within the ketones-responsive active area, β-hydroxybutyrate dehydrogenase may convert β-hydroxybutyrate and oxidized nicotinamide adenine dinucleotide (NAD⁺) into acetoacetate and reduced nicotinamide adenine dinucleotide (NADH), respectively. It is to be understood that the term "nicotinamide adenine dinucleotide (NAD)" includes a phosphate-bound form of the foregoing enzyme cofactors. That is, use of the term "NAD" herein refers to both NAD⁺ phosphate and NADH phosphate, specifically a diphosphate linking the two nucleotides, one containing an adenine nucleobase and the other containing a nicotinamide nucleobase. The NAD⁺/NADH enzyme cofactor aids in promoting the concerted enzymatic reactions disclosed herein. Once formed, NADH may undergo oxidation under diaphorase mediation, with the electrons transferred during this process providing the basis for ketone detection at the working electrode. Thus, there is a 1:1 molar correspondence between the amount of electrons transferred to the working electrode and the amount of β-hydroxybutyrate converted. Transfer of the electrons to the working electrode may take place under further mediation of an electron transfer agent, such as an osmium (Os) compound or similar transition metal complex, as described in additional detail below. Albumin may further be present as a stabilizer within the active area. The β-hydroxybutyrate dehydrogenase and the diaphorase may be covalently bonded to a polymer comprising the ketones-responsive active area. The NAD⁺ may or may not be covalently bonded to the polymer, but if the NAD⁺ is not covalently bonded, it may be physically retained within the ketones-responsive active area, such as with a mass transport limiting membrane overcoating the ketones-responsive active area, wherein the mass transport limiting membrane is also permeable to ketones.

Other suitable chemistries for enzymatically detecting ketones are shown in FIGS. 11B and 11C. In both instances, there is again a 1:1 molar correspondence between the amount of electrons transferred to the working electrode and the amount of β-hydroxybutyrate converted, thereby providing the basis for ketones detection.

As shown in FIG. 11B, β-hydroxybutyrate dehydrogenase (HBDH) may again convert β-hydroxybutyrate and NAD⁺ into acetoacetate and NADH, respectively. Instead of electron transfer to the working electrode being completed by diaphorase (see FIG. 11A) and a suitable redox mediator, the reduced form of NADH oxidase (NADHOx (Red)) undergoes a reaction to form the corresponding oxidized form (NADHOx (Ox)). NADHOx (Red) may then reform through a reaction with molecular oxygen to produce superoxide, which may undergo subsequent conversion to hydrogen peroxide under superoxide dismutase (SOD) mediation. The hydrogen peroxide may then undergo oxidation at the working electrode to provide a signal that may be correlated to the amount of ketones that were initially present. The SOD may be covalently bonded to a polymer in the ketones-responsive active area, according to various embodiments. Like the enzyme system shown in FIG. 11A, the β-hydroxybutyrate dehydrogenase and the NADH oxidase may be covalently bonded to a polymer in the ketones-responsive active area, and the NAD⁺ may or may not be covalently bonded to a polymer in the ketones-responsive active area. If the NAD⁺ is not covalently bonded, it may be physically retained within the ketones-responsive active area, with a membrane polymer promoting retention of the NAD⁺ within the ketones-responsive active area.

As shown in FIG. 11C, another enzymatic detection chemistry for ketones may utilize β-hydroxybutyrate dehydrogenase (HBDH) to convert β-hydroxybutyrate and NAD⁺ into acetoacetate and NADH, respectively. The electron transfer cycle in this case is completed by oxidation of NADH by 1,10-phenanthroline-5,6-dione to reform NAD⁺, wherein the 1,10-phenanthroline-5,6-dione subsequently transfers electrons to the working electrode. The 1,10-phenanthroline-5,6-dione may or may not be covalently bonded to a polymer within the ketones-responsive active area. Like the enzyme system shown in FIG. 11A, the β-hydroxybutyrate dehydrogenase may be covalently bonded to a polymer in the ketones-responsive active area, and the NAD⁺ may or may not be covalently bonded to a polymer in the ketones-responsive active area. Inclusion of an albumin in the active area may provide a surprising improvement in response stability. A suitable membrane polymer may promote retention of the NAD⁺ within the ketones-responsive active area.

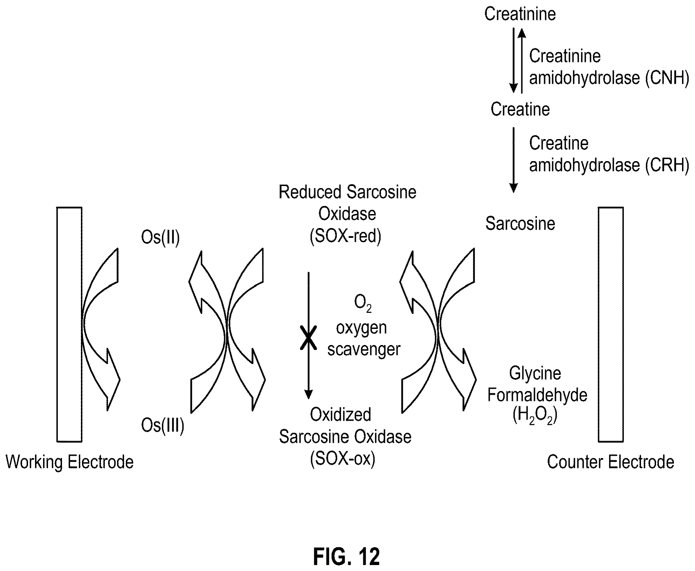

In some embodiments, the analyte sensors may further comprise a creatinine-responsive active area comprising an enzyme system that operates in concert to facilitate detection of creatinine. A suitable enzyme system that may be used for detecting creatinine in the analyte sensors disclosed herein is shown in FIG. 12 and described in further detail below. Additional details concerning enzyme systems responsive to creatinine may be found in commonly owned U.S. Patent Application 16/774,835 entitled "Analyte Sensors and Sensing Methods for Detecting Creatinine," filed on September 25, 2019, and published as U.S. Patent Application Publication _________.

As shown in FIG. 12, creatinine may react reversibly and hydrolytically in the presence of creatinine amidohydrolase (CNH) to form creatine. Creatine, in turn, may undergo catalytic hydrolysis in the presence of creatine amidohydrolase (CRH) to form sarcosine. Neither of these reactions produces a flow of electrons (*e*.*g*., oxidation or reduction) to provide a basis for electrochemical detection of the creatinine.

Referring still to FIG. 12, the sarcosine produced via hydrolysis of creatine may undergo oxidation in the presence of the oxidized form of sarcosine oxidase (SOX-ox) to form glycine and formaldehyde, thereby generating the reduced form of sarcosine oxidase (SOX-red) in the process. Hydrogen peroxide also may be generated in the presence of oxygen. The reduced form of sarcosine oxidase, in turn, may then undergo re-oxidation in the presence of the oxidized form of an electron transfer agent (*e*.*g*., an Os(III) complex), thereby producing the corresponding reduced form of the electron transfer agent (*e*.*g*., an Os(II) complex) and delivering a flow of electrons to the working electrode.

Oxygen may interfere with the concerted sequence of reactions used to detect creatinine in accordance with the disclosure above. Specifically, the reduced form of sarcosine oxidase may undergo a reaction with oxygen to reform the corresponding oxidized form of this enzyme but without exchanging electrons with the electron transfer agent. Although the enzymes all remain active when the reaction with oxygen occurs, no electrons flow to the working electrode. Without being bound by theory or mechanism, the competing reaction with oxygen is believed to result from kinetic effects. That is, oxidation of the reduced form of sarcosine oxidase with oxygen is believed to occur faster than does oxidation promoted by the electron transfer agent. Hydrogen peroxide is also formed in the presence of the oxygen.

The desired reaction pathway for facilitating detection of creatinine, shown in FIG. 12, may be encouraged by including an oxygen scavenger in proximity to the enzyme system. Various oxygen scavengers and dispositions thereof may be suitable, including oxidase enzymes such as glucose oxidase. Small molecule oxygen scavengers may also be suitable, but they may be fully consumed before the sensor lifetime is otherwise fully exhausted. Enzymes, in contrast, may undergo reversible oxidation and reduction, thereby affording a longer sensor lifetime. By discouraging oxidation of the reduced form of sarcosine oxidase with oxygen, the slower electron exchange reaction with the electron transfer agent may occur, thereby allowing production of a current at the working electrode. The magnitude of the current produced is proportional to the amount of creatinine that was initially reacted.

The oxygen scavenger used for encouraging the desired reaction pathway in FIG. 12 may be an oxidase enzyme in any embodiment of the present disclosure. Any oxidase enzyme may be used to promote oxygen scavenging in proximity to the enzyme system, provided that a suitable substrate for the enzyme is also present, thereby providing a reagent for reacting with the oxygen in the presence of the oxidase enzyme. Oxidase enzymes that may be suitable for oxygen scavenging in the present disclosure include, but are not limited to, glucose oxidase, lactate oxidase, xanthine oxidase, and the like. Glucose oxidase may be a particularly desirable oxidase enzyme to promote oxygen scavenging due to the ready availability of glucose in various bodily fluids. Reaction 1 below shows the enzymatic reaction promoted by glucose oxidase to afford oxygen clearing.

β -D-glucose + O₂ --→ D-glucono-1,5-lactone + H₂O₂ Reaction 1

The concentration of available lactate *in vivo* is lower than that of glucose, but still sufficient to promote oxygen scavenging.

Oxidase enzymes, such as glucose oxidase, may be positioned in any location suitable to promote oxygen scavenging in the analyte sensors disclosed herein. Glucose oxidase, for example, may be positioned upon the sensor tail such that the glucose oxidase is functional and/or non-functional for promoting glucose detection. When non-functional for promoting glucose detection, the glucose oxidase may be positioned upon the sensor tail such that electrons produced during glucose oxidation are precluded from reaching the working electrode, such as through electrically isolating the glucose oxidase from the working electrode.

In some embodiments, the analyte sensors may comprise a lactate-responsive active area comprising a lactate-responsive enzyme disposed upon the sensor tail. Suitable lactate-responsive enzymes may include, for example, lactate oxidase. Lactate oxidase or other lactate-responsive enzymes may be covalently bonded to a polymer comprising the lactate-responsive active area and exchange electrons with an electron transfer agent (*e*.*g*., an osmium (Os) complex or similar transition metal complex), which may also be covalently bonded to the polymer. Suitable electron transfer agents are described in further detail below. An albumin, such as human serum albumin, may be present in the lactate-responsive active area to stabilize the sensor response, as described in further detail in commonly owned U.S. Patent Application Publication 20190320947, which is incorporated herein by reference in its entirety. Lactate levels may vary in response to numerous environmental or physiological factors including, for example, eating, stress, exercise, sepsis or septic shock, infection, hypoxia, presence of cancerous tissue, or the like.

In some embodiments, the analyte sensors may comprise an active area responsive to pH. Suitable analyte sensors configured for determining pH are described in commonly owned U.S. Patent Application Publication 20200060592, which is incorporated herein by reference. Such analyte sensors may comprise a sensor tail comprising a first working electrode and a second working electrode, wherein a first active area located upon the first working electrode comprises a substance having pH-dependent oxidation-reduction chemistry, and a second active area located upon the second working electrode comprises a substance having oxidation-reduction chemistry that is substantially invariant with pH. By obtaining a difference between the first signal and the second signal, the difference may be correlated to the pH of a fluid to which the analyte sensor is exposed.

Two different types of active areas may be located upon a single working electrode, such as the carbon working electrodes discussed above, and spaced apart from one another. Each active area may have an oxidation-reduction potential, wherein the oxidation-reduction potential of the first active area is sufficiently separated from the oxidation-reduction potential of the second active area to allow independent production of a signal from one of the active areas. By way of non-limiting example, the oxidation-reduction potentials may differ by at least about 100 mV, or by at least about 150 mV, or by at least about 200 mV. The upper limit of the separation between the oxidation-reduction potentials is dictated by the working electrochemical window *in vivo.* By having the oxidation-reduction potentials of the two active areas sufficiently separated in magnitude from one another, an electrochemical reaction may take place within one of the two active areas (*i.e.,* within the first active area or the second active area) without substantially inducing an electrochemical reaction within the other active area. Thus, a signal from one of the first active area or the second active area may be independently produced at or above its corresponding oxidation-reduction potential (the lower oxidation-reduction potential) but below the oxidation-reduction potential of the other active area. A difference signal may allow the signal contribution from each analyte to be resolved.

Some or other embodiments of analyte sensors disclosed herein may feature two different active areas located upon the surface of separate working electrodes. Such analyte sensors may comprise a sensor tail comprising at least a first working electrode and a second working electrode, a first active area disposed upon a surface of the first working electrode, and a second active area responsive to a different analyte disposed upon a surface of the second working electrode. A membrane may overcoat at least one of the first active area and the second active area. The membrane may be a mass transport limiting membrane and may comprise a multi-component membrane where the membrane overcoats at least one of the active areas. The multi-component membrane may comprise a bilayer of two different membrane polymers or an admixture of two different membrane polymers, wherein one of the membrane polymers overcoats the other active area. Such multi-component membranes may be deposited by dip-coating techniques in non-limiting examples.

Other suitable mass transport limiting membranes may be deposited by *in situ* polymerization, such as *in situ* photopolymerization, as discussed in further detail hereinbelow.

An electron transfer agent may be present in any of the active areas disclosed herein. Suitable electron transfer agents may facilitate conveyance of electrons to the adjacent working electrode after one or more analytes undergoes an enzymatic oxidation-reduction reaction within the corresponding active area, thereby generating an electron flow that is indicative of the presence of a particular analyte. The amount of current generated is proportional to the quantity of analyte that is present. Depending on the sensor configuration used, the electron transfer agents in active areas responsive to different analytes may be the same or different. For example, when two different active areas are disposed upon the same working electrode, the electron transfer agent within each active area may be different (*e*.*g*., chemically different such that the electron transfer agents exhibit different oxidation-reduction potentials). When multiple working electrodes are present, the electron transfer agent within each active area may be the same or different, since each working electrode may be interrogated separately.

Suitable electron transfer agents may include electroreducible and electrooxidizable ions, complexes or molecules (*e*.*g*., quinones) having oxidation-reduction potentials that are a few hundred millivolts above or below the oxidation-reduction potential of the standard calomel electrode (SCE). According to some embodiments, suitable electron transfer agents may include low-potential osmium complexes, such as those described in U.S. Patents 6,134,461 and 6,605,200, which are incorporated herein by reference in their entirety. Additional examples of suitable electron transfer agents include those described in U.S. Patents 6,736,957, 7,501,053 and 7,754,093, the disclosures of each of which are incorporated herein by reference in their entirety. Other suitable electron transfer agents may comprise metal compounds or complexes of ruthenium, osmium, iron (*e*.*g*., polyvinylferrocene or hexacyanoferrate), or cobalt, including metallocene compounds thereof, for example. Suitable ligands for the metal complexes may also include, for example, bidentate or higher denticity ligands such as, for example, bipyridine, biimidazole, phenanthroline, or pyridyl(imidazole). Other suitable bidentate ligands may include, for example, amino acids, oxalic acid, acetylacetone, diaminoalkanes, or o-diaminoarenes. Any combination of monodentate, bidentate, tridentate, tetradentate, or higher denticity ligands may be present in a metal complex to achieve a full coordination sphere.

Active areas suitable for detecting any of the analytes disclosed herein may comprise a polymer to which the electron transfer agents are covalently bound. Any of the electron transfer agents disclosed herein may comprise suitable functionality to promote covalent bonding to the polymer within the active areas. Suitable examples of polymer-bound electron transfer agents may include those described in U.S. Patents 8,444,834, 8,268,143 and 6,605,201, the disclosures of which are incorporated herein by reference in their entirety. Suitable polymers for inclusion in the active areas may include, but are not limited to, polyvinylpyridines (*e.g.,* poly(4-vinylpyridine)), polyvinylimidazoles (*e.g.,* poly(1-vinylimidazole)), or any copolymer thereof. Illustrative copolymers that may be suitable for inclusion in the active areas include those containing monomer units such as styrene, acrylamide, methacrylamide, or acrylonitrile, for example. When two or more different active areas are present, the polymer within each active area may be the same or different.

Covalent bonding of the electron transfer agent to a polymer within an active area may take place by polymerizing a monomer unit bearing a covalently bonded electron transfer agent, or the electron transfer agent may be reacted with the polymer separately after the polymer has already been synthesized. A bifunctional spacer may covalently bond the electron transfer agent to the polymer within the active area, with a first functional group being reactive with the polymer (*e.g.,* a functional group capable of quaternizing a pyridine nitrogen atom or an imidazole nitrogen atom) and a second functional group being reactive with the electron transfer agent (*e*.*g*., a functional group that is reactive with a ligand coordinating a metal ion).

Similarly, one or more of the enzymes within the active areas may be covalently bonded to a polymer comprising an active area. When an enzyme system comprising multiple enzymes is present in a given active area, all of the multiple enzymes may be covalently bonded to the polymer in some embodiments, and in other embodiments, only a portion of the multiple enzymes may be covalently bonded to the polymer. For example, one or more enzymes comprising an enzyme system may be covalently bonded to the polymer and at least one enzyme may be non-covalently associated with the polymer, such that the non-covalently bonded enzyme is physically entrained within the polymer. Covalent bonding of the enzyme(s) to the polymer in a given active area may take place via a crosslinker introduced with a suitable crosslinking agent. Suitable crosslinking agents for reaction with free amino groups in the enzyme (*e*.*g*., with the free side chain amine in lysine) may include crosslinking agents such as, for example, polyethylene glycol diglycidyl ether (PEGDGE) or other polyepoxides, cyanuric chloride, N-hydroxysuccinimide, imidoesters, epichlorohydrin, or derivatized variants thereof. Suitable crosslinking agents for reaction with free carboxylic acid groups in the enzyme may include, for example, carbodiimides. The crosslinking of the enzyme to the polymer is generally intermolecular, but can be intramolecular in some embodiments. In particular embodiments, all of the enzymes within a given active area may be covalently bonded to a polymer.

The electron transfer agent and/or the enzyme(s) may be associated with the polymer in an active area through means other than covalent bonding as well. In some embodiments, the electron transfer agent and/or the enzyme(s) may be ionically or coordinatively associated with the polymer. For example, a charged polymer may be ionically associated with an oppositely charged electron transfer agent or enzyme(s). In still other embodiments, the electron transfer agent and/or the enzyme(s) may be physically entrained within the polymer without being bonded thereto. Physically entrained electron transfer agents and/or enzyme(s) may still suitably interact with a fluid to promote analyte detection without being substantially leached from the active areas.

The polymer within the active area(s) may be chosen such that outward diffusion of NAD⁺ or another cofactor not covalently bound to the polymer is limited. Limited outward diffusion of the cofactor may promote a reasonable sensor lifetime (days to weeks) while still allowing sufficient inward analyte diffusion to promote detection.

The active area(s) in the analyte sensors disclosed herein may comprise one or more discrete spots (*e*.*g*., one to about twenty spots, or even more discrete spots), which may range in size from about 0.01 mm² to about 1 mm² or about 0.05 mm² to about 0.3 mm², although larger or smaller individual spots within the active areas are also contemplated herein. When two different active areas are present, the number and/or size of individual spots may be the same or different for each type of active area.

It is also to be appreciated that the sensitivity (output current) of the analyte sensors toward a given analyte or combination of analytes may be varied by changing the coverage (area or size) of the active areas, the areal ratio of the active areas with respect to one another, the identity, thickness and/or composition of a mass transport limiting membrane overcoating the active areas. Variation of these parameters may be conducted readily by one having ordinary skill in the art once granted the benefit of the disclosure herein.

In more specific embodiments, analyte sensors of the present disclosure may comprise a sensor tail that is configured for insertion into a tissue. Suitable tissues are not considered to be particularly limited and are addressed in more detail above. Similarly, considerations for deploying a sensor tail at a particular position within a given tissue, such as a dermal layer of the skin, are addressed above.

In particular embodiments of the present disclosure, the mass transport limiting membrane overcoating one or more active areas may comprise a crosslinked polyvinylpyridine homopolymer or copolymer. The composition of the mass transport limiting membrane may be the same or different where the mass transport limiting membrane overcoats active areas of differing types. When the membrane composition varies at two different locations, the membrane may comprise a bilayer membrane or a homogeneous admixture of two different membrane polymers, one of which may be a crosslinked polyvinylpyridine or polyvinylimidazole homopolymer or copolymer. Suitable techniques for depositing a mass transport limiting membrane upon the active area(s) may include, for example, spray coating, painting, inkjet printing, screen printing, stenciling, roller coating, dip coating, the like, and any combination thereof. Dip coating techniques may be especially desirable for polyvinylpyridine and polyvinylimidazole polymers and copolymers.

Other suitable mass transport limiting membranes may be polymerized *in situ* upon a surface of one or more working electrodes. Particular examples of mass transport limiting membranes formable *in situ* may comprise a photopolymerized (photocured) mass transport limiting membrane. When multiple types of active areas are present, a photopolymerized mass transport limiting membrane may be formed selectively upon each type of active area. That is, a mass transport limiting membrane having a first portion may overcoat a first type of active area and a second portion differing in composition from the first portion may overcoat a second type of active area. The mass transport limiting membrane may be continuous or discontinuous between two or more active areas. More particularly, such photopolymerized mass transport limiting membranes may be formed by depositing one or more monomers upon the second face in contact with the one or more active areas and photopolymerizing the one or more monomers. Suitable deposition techniques for depositing the monomers include, for example, spraying, screen printing, or any combination thereof. Membrane chemistries that may be accessed through photopolymerization include, for example, acrylate polymers and copolymers, thiol-ene copolymers, or any combination thereof. Polymerization to form both of these types of membranes may occur under conditions that do not interfere with the sensing chemistry in the active area. Additional *in situ* crosslinking may also occur upon the surface of the working electrode(s) in the course of forming a mass transport limiting membrane *in situ.*

In non-limiting embodiments, mass transport limiting membranes suitable for incorporation in analyte sensors of the present disclosure may comprise a polymer or copolymer comprising one or more acrylate monomers. The term "acrylate monomer" refers to acrylic acid, methacrylic acid, or a derivative thereof. Monomers that may be either acrylates or methacrylates are designated herein as "(meth)acrylate monomers." That is, designation of a particular monomer as a "(meth)acrylate" refers to both the particular acrylate monomer and the corresponding methacrylate monomer form. Suitable acrylate monomers that may be present in the mass transport limiting membranes of the disclosure herein include, for example, 2-hydroxyethyl(meth)acrylate, poly(ethylene glycol) methyl ether (meth)acrylate, alkyl ether poly(ethylene glycol) (meth)acrylate, poly(ethylene glycol) (meth)acrylate, poly(propylene glycol) (meth)acrylate, alkyl ether poly(propylene glycol) (meth)acrylate, isobutyl (meth)acrylate, alkyl (meth)acrylates, 2-(trimethylsilyloxy)ethyl (meth)acrylate, 3-[tris(trimethlsiloxy)silyl]propyl (meth)acrylate, N,N-dimethylacrylamide, glycidyl (meth)acrylate, or any combination thereof. Glycidyl (meth)acrylate may be further functionalized in some cases. Crosslinkable acrylates such as di(ethylene glycol) di(meth)acrylate, poly(ethylene glycol) di(meth)acrylate, trimethylolpropane triacrylate, or any combination thereof may be present in combination with any of the foregoing (meth)acrylate monomers. Any molecule containing two or more alkene groups of sufficient reactivity may also serve as a crosslinker when forming the mass transport limiting membrane. The amount of the crosslinkable (meth)acrylate may be selected to afford a desired extent of crosslinking, such as a weight percentage of crosslinkable (meth)acrylate up to about 1 wt. % relative to the total mass of monomers. The choice of particular (meth)acrylate monomers, crosslinkable (meth)acrylate or similar polyene crosslinker, or any combination thereof and a ratio therebetween may be selected to afford sufficient permeability toward a given analyte of interest. For example, two or more (meth)acrylate monomers having differing levels of hydrophilicity and hydrophobicity may be selected, and the ratio thereof may be varied to afford particular membrane characteristics dependent upon water content and analyte hydrophilicity or hydrophobicity. Membranes comprising glycidyl (meth)acrylate may also be crosslinked with diol or triol crosslinkers such as ethylene glycol, propylene glycol, butanediol, hexanediol, glycerol, poly(ethylene glycol), poly(propylene glycol), or any combination thereof.

In addition, (meth)acrylamide, alkyl or dialkyl(meth)acrylamides, (meth)acrylic acid, or alkyl (meth)acrylate esters may be co-polymerized with any of the foregoing (meth)acrylate monomers or crosslinkable acrylates. Other olefinically unsaturated monomers may also be copolymerized with any of the above (meth)acrylate monomers. Other monomers that may be copolymerized include alpha olefins such as, for example, ethylene, propylene, butene, pentene, hexene, heptene, octene, nonene, decene, undecene, dodecene or mixtures thereof. Alpha olefins that are liquids at room temperature may be especially desirable. Such comonomers may be included to promote further tailoring of the physical properties of the resulting membrane. For example, one or more alpha olefin monomers may be added to increase the hydrophobicity of the membrane.
Some acrylate monomers may undergo photopolymerization or thermal polymerization in the absence of an initiator. Other acrylate monomers may undergo photopolymerization in the presence of a suitable initiator that generates a radical species when exposed to electromagnetic radiation, such as ultraviolet light (*e*.*g*., from a mercury lamp). In non-limiting embodiments, suitable photoinitiators may include, for example, 2,2-dimethoxy-2-phenylacetophenone, camphorquinone, azo compounds, and perioxides (*e*.*g*., benzoyl peroxide with N,N-dimethyltoluidine accelerator). Other suitable photoinitiators may include, but are not limited to Norrish Type I initiators (*e.g.,* 2,2-dimethoxy-2-phenylacetophenone, hydroxyacetophenones, aminoacetophenones, acetophenone phosphine oxides, and the like), Norrish Type II initiators (*e*.*g*., benzophenones, benzyl formates, thioxanthones, and the like, with an amine synergist promoter in some cases), phenol glyoxalic acid methyl ester, alpha-aminoketones, benzoyldiphenylphosphine oxides, or any combination thereof. CAROCUR, IRGACUR, and LUCIRIN photoinitiators from BASF are representative examples. Suitable photoinitiators may be present in an amount up to about 1 wt. % relative to the total mass of monomers.

Thiol-ene copolymers may also be suitably incorporated as a mass transport limiting membrane in the analyte sensors disclosed herein. Advantageously, thiol-ene polymers may be formed in air, in contrast to (meth)acrylate polymers, which may require the use of inert atmosphere for satisfactory polymerization to take place. Thiol-ene polymers may be accessed by reacting a monomer containing two or more thiol groups with a monomer containing two or more non-conjugated alkene groups. If needed, any of the above photoinitiators may be employed. Monomers with two thiol groups or two non-conjugated alkene groups may afford linear polymers. Monomers with three or more thiol groups or three or more non-conjugated alkene groups may afford variable extents of crosslinking. Non-crosslinkable monomers of either type may be used to decrease the crosslinking density, for example. Crosslinkable monomers, which may be tripodal or tetrapodal in nature, may have a variable length spacer between a central atom and a terminal thiol group or a terminal alkene group. The spacer length and the amount of crosslinkable monomer may impact the crosslinking density as well.

Particularly suitable monomers containing thiol groups may be tetrapodal, with each arm of the tetrapodal structure being terminated with a thiol group. Compounds 1 and 2 below are illustrative examples of monomers containing tetrapodally arranged thiol groups that may be suitable for use in forming mass transport limiting membranes through photopolymerization. 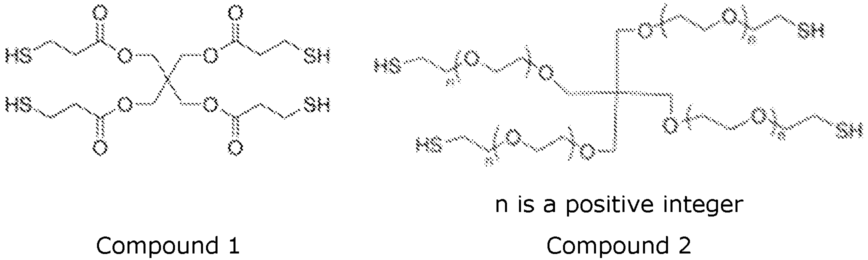 Compound 3 below, a dithiol, may be used to decrease the crosslinking density by introducing a linear portion into the thiol-ene polymer. Small dithiols such as ethanedithiol and propanedithiol may also be used in a like manner for decreasing the crosslinking density, as may polyetherdithiols larger than Compound 3. Trithiols may similarly be used for decreasing the crosslinking density.

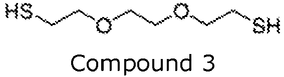

Particularly suitable ene monomers may be dipodal, tripodal, or tetrapodal, depending upon the desired crosslinking density. Compounds 4-6 are illustrative examples of dipodal, tripodal, and tetrapodal ene monomers, respectively, wherein A is a spacer group that is not particularly limited in structure and may optionally contain one or more heteroatoms. 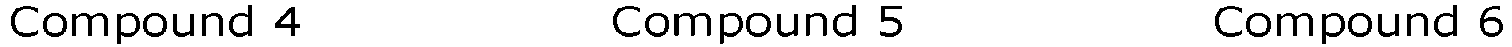 Compounds 7-10 below are illustrative examples of specific ene monomers that may be suitable for forming mass transport limiting membranes through *in situ* photopolymerization.

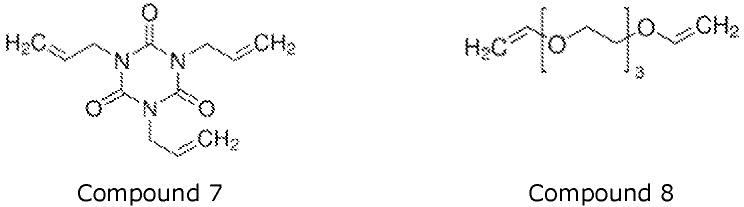

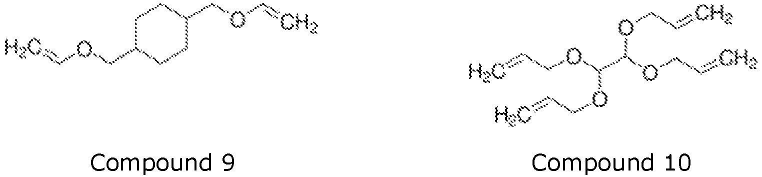

Photopolymerized mass transport limiting membranes may be present in analyte sensors comprising the carbon working electrode described hereinabove, or the photopolymerized mass transport limiting membranes may be present in analyte sensors comprising a conventional carbon working electrode or any other type of working electrode. In either case, the photopolymerized mass transport limiting membranes may be advantageous due to their ability to be readily tailored to meet particular application-specific needs. In addition, photopolymerized mass transport limiting membranes may be accessed by deposition of readily dispensed monomers, which may be deposited in one or more specific locations by spraying or screen printing techniques. As a result of the deposition specificity, photopolymerized mass transport limiting membranes having different compositions may overcoat active areas of different types. Alternately, a photopolymerized mass transport limiting membrane may overcoat one type of active area while a second type of active area may be overcoated with a polyvinylpyridine or polyvinylimidazole polymer or copolymer deposited through dip coating.

Accordingly, some analyte sensors of the present disclosure may comprise: a first working electrode, one or more first active areas disposed upon the first working electrode and that are responsive to a first analyte, and a first photopolymerized mass transport limiting membrane formed directly upon the first working electrode and overcoating at least one of the one or more first active areas. The first working electrode may be a first carbon electrode, such as the carbon electrodes described in more detail above. Conventional carbon working electrodes may also be suitable. In non-limiting embodiments, the first active area may comprise an analyte responsive enzyme or enzyme system. Any of the analytes discussed in more detail hereinabove may be assayed by the first active area.

The analyte sensors may also comprise a second working electrode, and one or more second active areas disposed upon the second working electrode, which are responsive to a second analyte different from the first analyte. The second working electrode may be a second carbon electrode, such as the carbon electrodes described in more detail above or a conventional carbon working electrode may also be suitable. A second mass transport limiting membrane may overcoat at least the one or more second active areas and differ in composition from the first photopolymerized mass transport limiting membrane. The second mass transport limiting membrane may comprise a dip-coated polyvinylpyridine or polyvinylimidazole polymer or copolymer or, more desirably, a second photopolymerized mass transport limiting membrane differing in composition from the first mass transport limiting membrane. The first photopolymerized mass transport limiting membrane and/or the second photopolymerized mass transport limiting membrane may be discontinuous, such that the membrane does not extend between adjacent spots of a given active area or between two different active areas configured for detecting different analytes.

Accordingly, methods for forming analyte sensors comprising a photopolymerized mass transport limiting membrane may comprise: providing an analyte sensor comprising at least a first working electrode having one or more first active areas disposed thereon, in which the one or more first active areas are responsive to a first analyte; applying one or more first monomers upon the first working electrode in contact with the one or more first active areas; and polymerizing the one or more first monomers *in situ* upon the first working electrode, such as through photopolymerization, to form a first mass transport limiting membrane overcoating at least the one or more first active areas. In non-limiting embodiments, photopolymerizing may comprise exposing the one or more first monomers (e.g., a mixture of a first monomer and a second monomer) to ultraviolet light and optionally a photoinitiator. When the analyte sensors further comprise a second working electrode, the methods may further comprise: applying one or more second monomers upon the second working electrode in contact with one or more second active areas; and polymerizing the one or more second monomers upon the second working electrode, such as through photopolymerization, to form a second mass transport limiting membrane overcoating at least the one or more second active areas. The second mass transport limiting membrane may have the same composition or differ in composition from the first mass transport limiting membrane. In various embodiments, the first mass transport limiting membrane and the second mass transport limiting membrane may both be photopolymerized.

The analyte sensors may be prepared in aggregate using an electrode material (*e*.*g*., carbon) having a plurality of active areas disposed thereon. Once the one or more first monomers have been applied to the active areas, such as through syringe deposition of a solution comprising the one or more first monomers, polymerization may take place as specified above. The solution comprising the one or more first monomers may be deposited at about 100-200 nL in non-limiting examples. After polymerization takes place, such as with ultraviolet photoirradiation at 365 nm and 1-30 mW/cm² irradiation power for up to 5 minutes, individual analyte sensors may be singulated by laser cutting the electrode material, and cutting through the mass transport limiting membrane in some instances.

Embodiments disclosed herein include:
A. Analyte sensors having a carbon working electrode. The analyte sensors comprise: a carbon working electrode comprising: a dielectric substrate, one or more apertures extending through the dielectric substrate, the one or more apertures each being filled with a carbon conductor pillar extending between a first face and a second face of the dielectric substrate, a carbon conductor coating disposed upon the first face of the dielectric substrate in direct contact with each carbon conductor pillar, and a dielectric coating disposed upon the carbon conductor coating; one or more active areas located upon the second face of the dielectric substrate that are in electrical communication with each of the carbon conductor pillars, the one or more active areas being responsive to an analyte; and a mass transport limiting membrane overcoating at least the one or more active areas.
B. Methods for producing an analyte sensor with a carbon working electrode. The methods comprise: providing a dielectric substrate having one or more apertures extending therethrough between a first face and a second face; filling the one or more apertures with a carbon conductor to form carbon conductor pillars therein and depositing a carbon conductor coating on the first face of the dielectric substrate, the carbon conductor coating being in direct contact with each carbon conductor pillar; depositing a dielectric coating upon the carbon conductor coating; forming one or more active areas upon the second face of the dielectric substrate in electrical communication with the carbon conductor pillars within the one or more apertures, the one or more active areas being responsive to an analyte; and depositing a mass transport limiting membrane upon at least the one or more active areas.
C. Analyte sensors having a mass transport limiting membrane. The analyte sensors comprise: a first working electrode; one or more first active areas disposed upon the first working electrode, the one or more first active areas being responsive to a first analyte; and a first photopolymerized mass transport limiting membrane formed directly upon the first working electrode and overcoating at least the one or more first active areas.
D. Methods for producing an analyte sensor having a mass transport limiting membrane. The methods comprise: providing an analyte sensor comprising at least a first working electrode having one or more first active areas disposed thereon, the one or more first active areas being responsive to a first analyte; applying one or more first monomers upon the first working electrode in contact with the one or more first active areas; and polymerizing the one or more first monomers *in situ* upon the first working electrode to form a first mass transport limiting membrane overcoating at least the one or more first active areas.

Each of embodiments A-D may have one or more of the following additional elements in any combination:
Element 1: wherein the one or more apertures comprise multiple vias extending through the dielectric substrate.
Element 2: wherein each via has an active area disposed directly upon the carbon conductor pillar located therein.
Element 3: wherein the analyte sensor further comprises a carbon conductor strip located upon the second face of the dielectric substrate and overcoating the multiple vias; wherein the one or more active areas are disposed directly upon the carbon conductor strip.
Element 4: wherein the one or more apertures comprise a slot extending through the dielectric substrate.
Element 5: wherein multiple active areas are disposed directly upon the carbon conductor pillar located within the slot.
Element 6: wherein the one or more active areas comprise one or more analyte-responsive enzymes.
Element 7: wherein the mass transport limiting membrane is a photopolymerized mass transport limiting membrane.
Element 8: wherein the photopolymerized mass transport limiting membrane is formed selectively on the one or more active areas.
Element 9: wherein the mass transport limiting membrane comprises an acrylate polymer or copolymer, a thiol-ene copolymer, or any combination thereof.
Element 10: wherein the carbon working electrode is disposed upon a sensor tail configured for insertion in a tissue.
Element 11: wherein the method further comprises depositing a carbon conductor strip upon the second face of the dielectric substrate, the carbon conductor strip overcoating the multiple vias; wherein the one or more active areas are disposed directly upon the carbon conductor strip.
Element 12: wherein the mass transport limiting membrane is deposited by dip coating.
Element 13: wherein the mass transport limiting membrane is polymerized *in situ* upon the one or more active areas.
Element 14: wherein the mass transport limiting membrane is a photopolymerized mass transport limiting membrane and is formed by depositing one or more monomers upon the second face of the dielectric substrate in contact with the one or more active areas and photopolymerizing the one or more monomers.
Element 15: wherein the photopolymerized mass transport limiting membrane comprises an acrylate polymer or copolymer, a thiol-ene copolymer, or any combination thereof.
Element 16: wherein the first photopolymerized mass transport limiting membrane comprises an acrylate polymer or copolymer, a thiol-ene copolymer, or any combination thereof.
Element 17: wherein the analyte sensor further comprises a second working electrode; one or more second active areas disposed upon the second working electrode, the one or more second active areas being responsive to a second analyte different from the first analyte; and a second mass transport limiting membrane overcoating at least the one or more second active areas and differing in composition from the first photopolymerized mass transport limiting membrane.
Element 18: wherein the second mass transport limiting membrane is a second photopolymerized mass transport limiting membrane differing in composition from the first photopolymerized mass transport limiting membrane.
Element 19: wherein the first photopolymerized mass transport limiting membrane is crosslinked.
Element 20: wherein the first photopolymerized mass transport limiting membrane is discontinuous.
Element 21: wherein the first photopolymerized mass transport limiting membrane is disposed substantially upon the one or more active areas.
Element 22: wherein the one or more first active areas comprise one or more analyte-responsive enzymes.
Element 23: wherein the first working electrode is disposed upon a sensor tail configured for insertion in a tissue.
Element 24: wherein the first mass transport limiting membrane is a photopolymerized mass transport limiting membrane formed by photopolymerizing the one or more first monomers.
Element 25: wherein applying the one or more first monomers comprises screen printing the one or more first monomers, spraying the one or more first monomers, or any combination thereof.
Element 26: wherein the analyte sensor further comprises a second working electrode having one or more second active areas disposed thereon, the one or more second active areas being responsive to a second analyte different from the first analyte, and the method further comprises: applying one or more second monomers upon the second working electrode in contact with the one or more second active areas; and polymerizing the one or more second monomers *in situ* upon the second working electrode to form a second mass transport limiting membrane overcoating at least the one or more second active areas, the second mass transport limiting membrane differing in composition from the first mass transport limiting membrane.
Element 27: wherein the second mass transport limiting membrane is a photopolymerized mass transport limiting membrane formed by photopolymerizing the one or more second monomers.
Element 28: wherein the first mass transport limiting membrane is discontinuous.
Element 29: wherein the first mass transport limiting membrane is formed selectively on the one or more first active areas.

By way of non-limiting example, exemplary combinations applicable to A include, but are not limited to: 1 and 2; 1 and 3; 1-3; 1 and 6; 1 and 7; 1 and 8; 1 and 9; 1 and 10; 4 and 5; 4 and 6; 4-6; 4 and 7; 4, 5 and 7; 4 and 8; 4, 5 and 8; 4 and 9; 4, 5 and 9; 4 and 10; and 4, 5 and 10. Exemplary combinations applicable to B include, but are not limited to: 1 and 2; 1 and 11; 1, 2 and 11; 1 and 6; 1 and 7; 1 and 8; 1 and 9; 1 and 10; 4 and 5; 4 and 6; 4-6; 4 and 7; 4, 5 and 7; 4 and 8; 4, 5 and 8; 4 and 9; 4, 5 and 9; 4 and 10; 4, 5 and 10; 1 and 12; 1, 2 and 12; 1 and 13; 1, 2 and 13; 1, 2, 11 and 13; 4 and 12; 4, 5 and 12; 4 and 13; 4, 5 and 13; 1, 13 and 14; 4, 13 and 14; 1 and 13-15; and 4 and 13-15. Exemplary combinations applicable to C include, but are not limited to: 16 and 17; 16-18; 16 and 19; 16 and 20; 16 and 21; 16 and 22; 16 and 23; 17 and 18; 17 and 19; 17 and 20; 17 and 21; 17 and 22; 17 and 23; 20 and 21; 20 and 22; 20 and 23; 21 and 22; 21 and 23; and 22 and 23. Exemplary combinations applicable to D include, but are not limited to, 16 and 24; 16, 24 and 25; 24 and 25; 16, 24 and 26; 24 and 26; 24, 26 and 27; 16, 24 and 28; 24 and 28; 16, 24, 26 and 27; 24 and 29; 16, 24 and 29; 26 and 27; 26 and 28; 26-28; 26 and 29; 26, 27 and 29; and 28 and 29.

To facilitate a better understanding of the embodiments described herein, the following examples of various representative embodiments are given. In no way should the following examples be read to limit, or to define, the scope of the invention.

### EXAMPLES

**General Procedure for Sensor Fabrication:** Appropriate sensing layer chemistry was first deposited onto a working electrode (*e*.*g*., a carbon working electrode) as a group of separate dots or a slot with an area of about 0.05 to 0.3 mm². Deposition of the sensing layer chemistry was usually carried out with a piezoelectric deposition device. A micro-syringe was then used to manually deposit approximately 100-200 nL monomer solution containing monomer(s), crosslinkers and initiators over the working electrode area of about 1.5 mm x 2.5 mm. The resulting construct was then transferred into an argon-filled chamber containing a UV lamp radiating at 365 nm. The mass transport limiting membrane was then photopolymerized at a UV power from 1 to 30 mW/cm² for up to 5 minutes. Individual sensors were then singulated by laser cutting.

Active areas for various analytes utilized a poly(vinylpyridine)-bound transition metal complex having the structure shown below in Formula 11 as an electron transfer agent. Further details concerning this transition metal complex and electron transfer therewith are provided in commonly owned U.S. Patent 6,605,200, which was incorporated by reference above. The subscripts for each monomer represent illustrative atomic ratios and are not indicative of any particular monomer ordering.

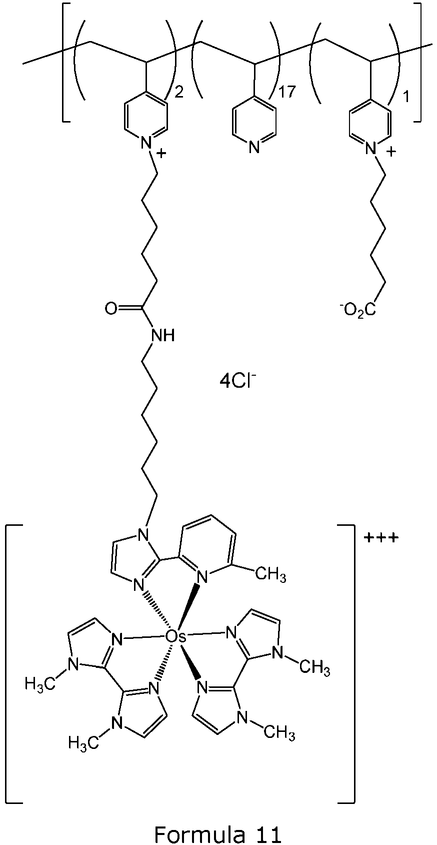

**Example 1: Glucose Sensors Overcoated with a Photopolymerized Membrane.** Glucose-responsive active areas were deposited using the formulation shown in Table 1 below, which was formulated in 10 mM HEPES buffer (pH = 8.05).

**Table 1**

| **Component** | **Concentration** |
|---|---|
| Formula 11 Polymer | 20.4 mg/mL |
| Glucose Oxidase | 24.6 mg/mL |
| PEGDGE400 | 7.5 mg/mL |

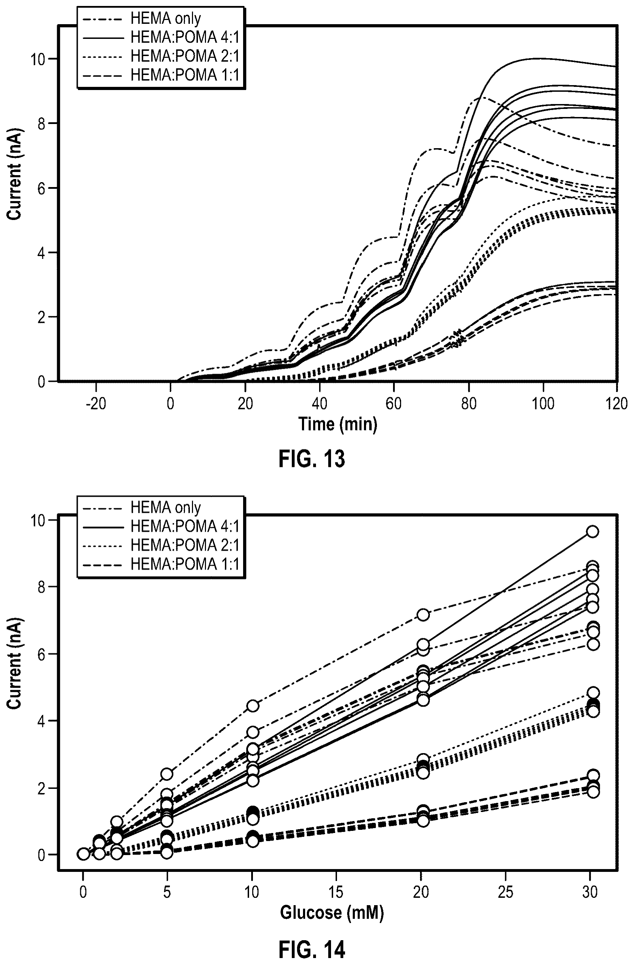

A total of 12 nL of the formulation in Table 1 was deposited upon a carbon working electrode using a piezoelectric dispensing system to form a glucose-responsive active area having an area of about 0.1 mm². A 200 nL volume of 2-hydroxyethyl methacrylate (HEMA) and poly(propylene glycol) methacryate (POMA) was then deposited upon the active area and polymerized at various molar ratios of monomer in the presence of 1 wt. % 2,2-dimethoxy-2-phenylacetophenone photoinitiator and 1 wt. % di(ethylene glycol) dimethacrylate crosslinker. Polymerization and crosslinking took place at a UV irradiation wavelength of 365 nm and a power of 6 mW/cm² under argon for 5 minutes. The sensors were then singulated to form a 0.7 mm wide tail by laser cutting through the photopolymerized membrane. FIG. 13 shows a plot of sensor response for several glucose sensors overcoated with a mass transport limiting membrane formed from HEMA:POMA at various ratios, following addition of 1 M glucose stock solution in various amounts to achieve a range of glucose concentrations in PBS at 33°C. FIG. 14 shows a corresponding plot of sensor response as a function of glucose concentration. Analyte sensors having a HEMA:POMA membrane exhibited an average drift of less than 10% over 14 days, whereas a membrane comprising HEMA alone drifted nearly 100% (data not shown). HEMA:POMA ratios of 2:1 and 4:1 afforded a good balance of signal, response linearity and extended response stability.

**Example 2: Ketone Sensors Overcoated with a HEMA-POMA Membrane.** Ketones-responsive active areas were deposited using the formulation shown in Table 2 below, which was formulated in 10 mM MES buffer (pH = 5.5).

**Table 2**

| **Component** | **Concentration** |
|---|---|
| Formula 11 Polymer | 8 mg/mL |
| β-Hydroxybutyrate Dehydrogenase | 8 mg/mL |
| Albumin | 8 mg/mL |
| NAD⁺ | 8 mg/mL |
| Diaphorase | 4 mg/mL |
| PEGDGE400 | 4 mg/mL |

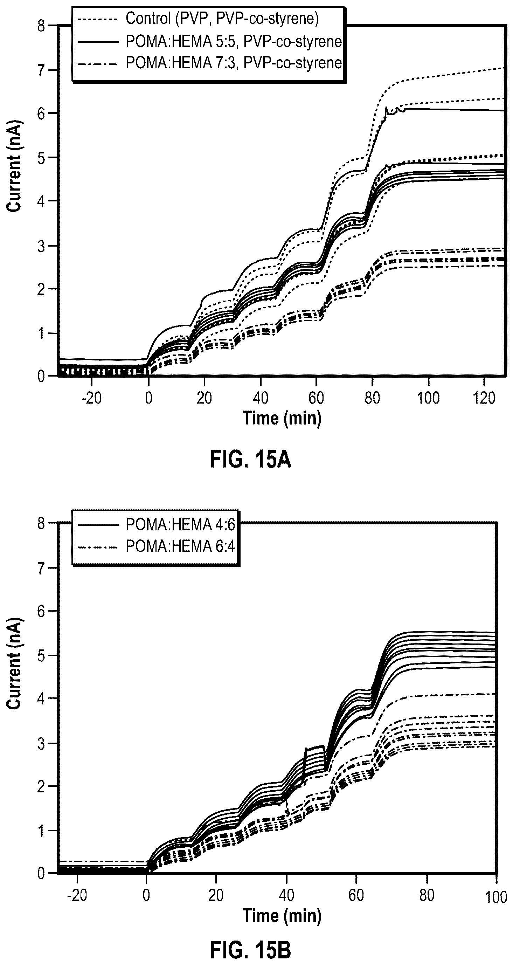

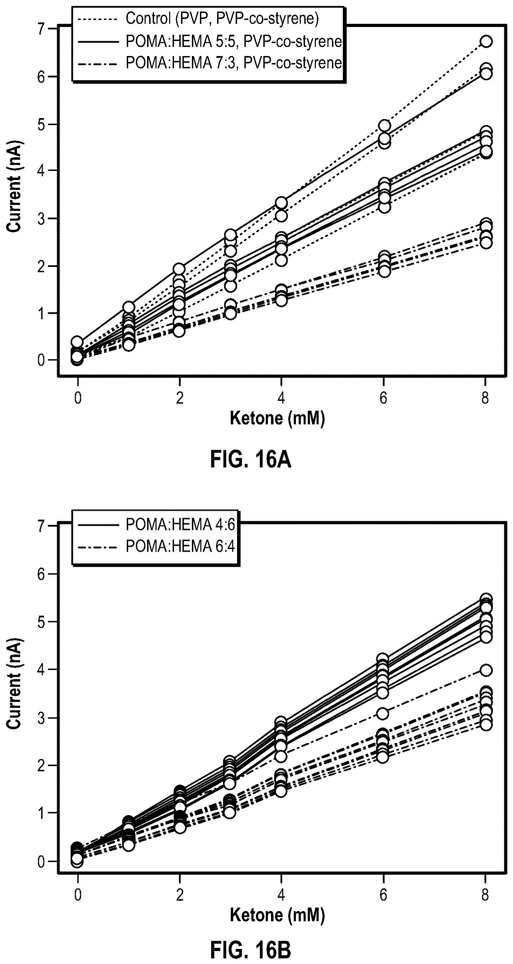

A total of 12 nL of the formulation in Table 2 was deposited upon a carbon working electrode using a piezoelectric dispensing system to form a ketones-responsive active area having an area of about 0.1 mm². A 200 nL volume of 2-hydroxyethyl methacrylate (HEMA) and poly(propylene glycol) methacryate (POMA) was then deposited upon the active area and polymerized at various molar ratios of monomer in the presence of 1 wt. % 2,2-dimethoxy-2-phenylacetophenone photoinitiator and 1 wt. % di(ethylene glycol) dimethacrylate crosslinker. Polymerization and crosslinking took place at a UV irradiation wavelength of 365 nm and a power of 6 mW/cm² under argon for 5 minutes. The sensors were then singulated to form a 0.7 mm wide tail by laser cutting through the photopolymerized membrane. A PVP-co-styrene layer was then formed upon the HEMA-POMA copolymer by dip coating the sensor tail. FIGS. 15A and 15B show plots of sensor response for several ketone sensors overcoated with a mass transport limiting membrane formed from HEMA:POMA at various ratios, following addition of 1 M ketones stock solution in various amounts to achieve a range of ketone concentrations in PBS at 33°C. FIGS. 16A and 16B show corresponding plots of sensor response as a function of ketone concentration. A HEMA:POMA ratio of 4:6 afforded a good balance of signal and response linearity. Sensor performance comparable to a dip-coated PVP/PVP-co-styrene mass transport limiting membrane control, which affords good performance when assaying ketones (U.S. Patent Application 16/774,835, referenced above), was achieved. The sensor response changed by about 2.4% over two weeks of measurement at this HEMA:POMA ratio (data not shown).

**Example 3: Crosslinking Density Variation within a Thiol-Ene Polymer System.** 2,2'-(ethane-1,2-diylbis(oxy))bis(ethane-1-thiol) (EDDEE) and 2-(5-mercapto-3-oxopentyl)-2-(((3-mercaptopropanoyl)oxy)methyl)propane-1,3-diyl bis(3-mercaptopropanoate) (PETMP) were combined in various molar ratios and photopolymerized with tri(ethylene glycol) divinyl ether (TEGDVE), as specified in Table 3 below. The molar ratio of the two thiols to TEGDVE was maintained at 1:1 in all instances. Polymerization was conducted by exposing the monomer mixture to a 365 nm UV light at a power density of 6 mW/cm² for 5 minutes under air. Table 3 also summarizes the resulting polymer properties obtained.

**Table 3**

| **Entry** | **EDDEE:PETMP Molar Ratio** | **Appearance** | **Water Content** |
|---|---|---|---|
| 1 | EDDEE only | Viscous liquid | N/A |
| 2 | 999:1 | Viscous liquid | N/A |
| 3 | 499:1 | Viscous liquid | N/A |
| 4 | 199:1 | Viscous liquid | N/A |
| 5 | 99:1 | Tacky gel | N/A |
| 6 | 49:1 | Soft solid | 10.4% |
| 7 | 19:1 | Solid | 10.2% |
| 8 | 9:1 | Solid | 6.5% |
| 9 | 4:1 | Solid | 4.4% |
| 10 | 2:1 | Solid | 3.7% |
| 11 | 1:1 | Solid | 7.0% |
| 12 | PETMP only | Hard solid | 6.2% |

Entries 1-12 in Table 1 are ranked in approximate order of crosslinking density, from lowest to highest. Crosslinking density was evaluated qualitatively by the polymer appearance, as well as semi-quantitatively by the amount of water absorbed by the polymer. The water content was measured by the ratio of the difference between the dry polymer mass and the wet polymer mass after soaking a piece of polymer in water for 8 hours) to the wet polymer mass. As shown in Table 3, more tetrafunctional thiol PETMP resulted in higher crosslinking density values. This example demonstrates that it is possible to adjust the permeability of the thiol-ene polymer system to account for the size of different analytes. For an aqueous system, higher water content of a polymer usually indicates a higher permeability value.

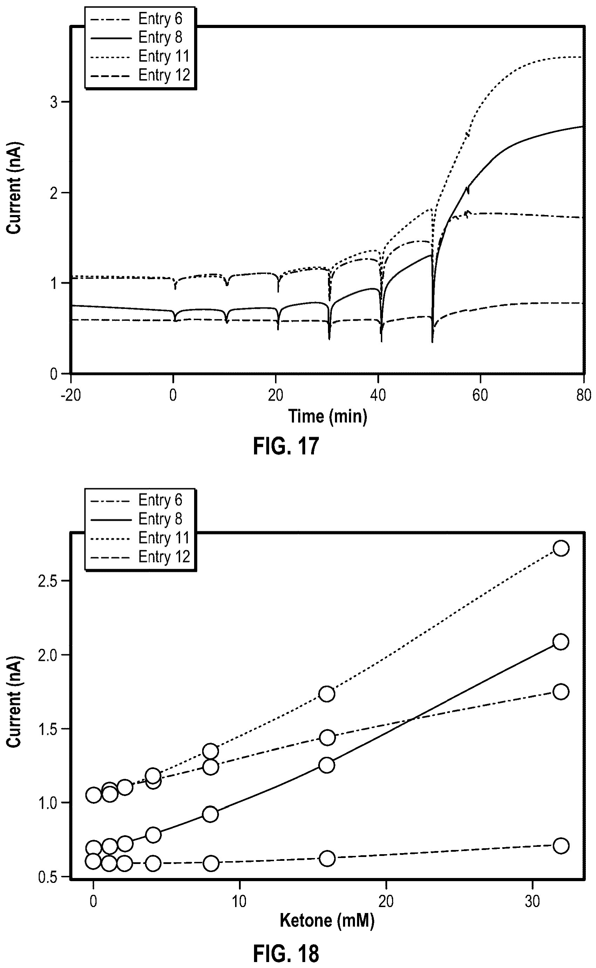

**Example 4: Ketone Sensors Overcoated with a Thiol-Ene Membrane.** Ketone sensors were fabricated as in Example 2 and overcoated with the thiol-ene copolymers from Entries 6, 8, 11 and 12 of Table 3. Photopolymerization was carried out as in Example 3. The sensors were then singulated to form a 0.7 mm wide tail by laser cutting through the photopolymerized membrane. A PVP-co-styrene layer was then formed upon the thiol-ene copolymer by dip coating the sensor tail. FIG. 17 shows a plot of sensor response for several ketone sensors overcoated with a mass transport limiting membrane formed from a thiol-ene polymer at various ratios, following addition of 1 M ketones stock solution in various amounts to achieve a range of ketone concentrations in PBS at 33°C. FIG. 18 shows a corresponding plot of sensor response as a function of ketone concentration. In general, the sensor response increased as the crosslinking density decreased, except for the membrane formed from Entry 6, which was a tacky membrane.

Unless otherwise indicated, all numbers expressing quantities and the like in the present specification and associated claims are to be understood as being modified in all instances by the term "about." Accordingly, unless indicated to the contrary, the numerical parameters set forth in the specification and attached claims are approximations that may vary depending upon the desired properties sought to be obtained by the embodiments of the present invention. At the very least, and not as an attempt to limit the application of the doctrine of equivalents to the scope of the claim, each numerical parameter should at least be construed in light of the number of reported significant digits and by applying ordinary rounding techniques.

One or more illustrative embodiments incorporating various features are presented herein. Not all features of a physical implementation are described or shown in this application for the sake of clarity. It is understood that in the development of a physical embodiment incorporating the embodiments of the present invention, numerous implementation-specific decisions must be made to achieve the developer's goals, such as compliance with system-related, business-related, government-related and other constraints, which vary by implementation and from time to time. While a developer's efforts might be time-consuming, such efforts would be, nevertheless, a routine undertaking for those of ordinary skill in the art and having benefit of this disclosure.

While various systems, tools and methods are described herein in terms of "comprising" various components or steps, the systems, tools and methods can also "consist essentially of" or "consist of" the various components and steps.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Therefore, the disclosed systems, tools and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems, tools and methods illustratively disclosed herein may suitably be practiced in the absence of any element that is not specifically disclosed herein and/or any optional element disclosed herein. While systems, tools and methods are described in terms of "comprising," "containing," or "including" various components or steps, the systems, tools and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

### CLAUSES:

1. An analyte sensor comprising:
   a carbon working electrode comprising:
      a dielectric substrate,
      one or more apertures extending through the dielectric substrate, the one or more apertures each being filled with a carbon conductor pillar extending between a first face and a second face of the dielectric substrate,
      a carbon conductor coating disposed upon the first face of the dielectric substrate in direct contact with each carbon conductor pillar, and
      a dielectric coating disposed upon the carbon conductor coating;
   one or more active areas located upon the second face of the dielectric substrate that are in electrical communication with each of the carbon conductor pillars, the one or more active areas being responsive to an analyte; and
   a mass transport limiting membrane overcoating at least the one or more active areas.
2. The analyte sensor of clause 1, wherein the one or more apertures comprise multiple vias extending through the dielectric substrate.
3. The analyte sensor of clause 2, wherein each via has an active area disposed directly upon the carbon conductor pillar located therein.
4. The analyte sensor of clause 2, further comprising:
   a carbon conductor strip located upon the second face of the dielectric substrate and overcoating the multiple vias;
   wherein the one or more active areas are disposed directly upon the carbon conductor strip.
5. The analyte sensor of clause 1, wherein the one or more apertures comprise a slot extending through the dielectric substrate.
6. The analyte sensor of clause 5, wherein multiple active areas are disposed directly upon the carbon conductor pillar located within the slot.
7. The analyte sensor of clause 1, wherein the one or more active areas comprise one or more analyte-responsive enzymes.
8. The analyte sensor of clause 1, wherein the mass transport limiting membrane is a photopolymerized mass transport limiting membrane.
9. The analyte sensor of clause 8, wherein the photopolymerized mass transport limiting membrane is formed selectively on the one or more active areas.
10. The analyte sensor of clause 9, wherein the mass transport limiting membrane comprises an acrylate polymer or copolymer, a thiol-ene copolymer, or any combination thereof.
11. The analyte sensor of clause 1, wherein the carbon working electrode is disposed upon a sensor tail configured for insertion in a tissue.
12. A method comprising:
   providing a dielectric substrate having one or more apertures extending therethrough between a first face and a second face;
   filling the one or more apertures with a carbon conductor to form carbon conductor pillars therein and depositing a carbon conductor coating on the first face of the dielectric substrate, the carbon conductor coating being in direct contact with each carbon conductor pillar;
   depositing a dielectric coating upon the carbon conductor coating;
   forming one or more active areas upon the second face of the dielectric substrate in electrical communication with the carbon conductor pillars within the one or more apertures, the one or more active areas being responsive to an analyte; and
   depositing a mass transport limiting membrane upon at least the one or more active areas.
13. The method of clause 12, wherein the one or more apertures comprise multiple vias extending through the dielectric substrate.
14. The method of clause 13, wherein each via has an active area disposed directly upon the carbon conductor pillar located therein.
15. The method of clause 13, further comprising:
   depositing a carbon conductor strip upon the second face of the dielectric substrate, the carbon conductor strip overcoating the multiple vias;
   wherein the one or more active areas are disposed directly upon the carbon conductor strip.
16. The method of clause 12, wherein the one or more apertures comprise a slot extending through the dielectric substrate.
17. The method of clause 16, wherein multiple active areas are disposed directly upon the carbon conductor pillar located within the slot.
18. The method of clause 12, wherein the mass transport limiting membrane is deposited by dip coating.
19. The method of clause 12, wherein the mass transport limiting membrane is polymerized *in situ* upon the one or more active areas.
20. The method of clause 19, wherein the mass transport limiting membrane is a photopolymerized mass transport limiting membrane and is formed by depositing one or more monomers upon the second face of the dielectric substrate in contact with the one or more active areas and photopolymerizing the one or more monomers.
21. The method of clause 20, wherein the photopolymerized mass transport limiting membrane comprises an acrylate polymer or copolymer, a thiol-ene copolymer, or any combination thereof.
22. An analyte sensor comprising:
   a first working electrode;
   one or more first active areas disposed upon the first working electrode, the one or more first active areas being responsive to a first analyte; and
   a first photopolymerized mass transport limiting membrane formed directly upon the first working electrode and overcoating at least the one or more first active areas.
23. The analyte sensor of clause 22, wherein the first photopolymerized mass transport limiting membrane comprises an acrylate polymer or copolymer, a thiol-ene copolymer, or any combination thereof.
24. The analyte sensor of clause 22, further comprising:
   a second working electrode;
   one or more second active areas disposed upon the second working electrode, the one or more second active areas being responsive to a second analyte different from the first analyte; and
   a second mass transport limiting membrane overcoating at least the one or more second active areas and differing in composition from the first photopolymerized mass transport limiting membrane.
25. The analyte sensor of clause 24, wherein the second mass transport limiting membrane is a second photopolymerized mass transport limiting membrane differing in composition from the first photopolymerized mass transport limiting membrane.
26. The analyte sensor of clause 22, wherein the first photopolymerized mass transport limiting membrane is crosslinked.
27. The analyte sensor of clause 22, wherein the first photopolymerized mass transport limiting membrane is discontinuous.
28. The analyte sensor of clause 27, wherein the first photopolymerized mass transport limiting membrane is disposed substantially upon the one or more active areas.
29. The analyte sensor of clause 22, wherein the one or more first active areas comprise one or more analyte-responsive enzymes.
30. The analyte sensor of clause 22, wherein the first working electrode is disposed upon a sensor tail configured for insertion in a tissue.
31. A method comprising:
   providing an analyte sensor comprising at least a first working electrode having one or more first active areas disposed thereon, the one or more first active areas being responsive to a first analyte;
   applying one or more first monomers upon the first working electrode in contact with the one or more first active areas; and
   polymerizing the one or more first monomers *in situ* upon the first working electrode to form a first mass transport limiting membrane overcoating at least the one or more first active areas.
32. The method of clause 31, wherein the first mass transport limiting membrane is a photopolymerized mass transport limiting membrane formed by photopolymerizing the one or more first monomers.
33. The method of clause 32, wherein the first photopolymerized mass transport limiting membrane comprises an acrylate polymer or copolymer, a thiol-ene copolymer, or any combination thereof.
34. The method of clause 31, wherein applying the one or more first monomers comprises screen printing the one or more first monomers, spraying the one or more first monomers, or any combination thereof.
35. The method of clause 31, wherein the analyte sensor further comprises a second working electrode having one or more second active areas disposed thereon, the one or more second active areas being responsive to a second analyte different from the first analyte, the method further comprising:
   applying one or more second monomers upon the second working electrode in contact with the one or more second active areas; and
   polymerizing the one or more second monomers *in situ* upon the second working electrode to form a second mass transport limiting membrane overcoating at least the one or more second active areas, the second mass transport limiting membrane differing in composition from the first mass transport limiting membrane.
36. The method of clause 35, wherein the second mass transport limiting membrane is a photopolymerized mass transport limiting membrane formed by photopolymerizing the one or more second monomers.
37. The method of clause 31, wherein the first mass transport limiting membrane is discontinuous.
38. The method of clause 37, wherein the first mass transport limiting membrane is formed selectively on the one or more first active areas.

## Claims

1. An analyte sensor comprising:
a first working electrode;
one or more first active areas disposed upon the first working electrode, the one or more first active areas being responsive to a first analyte; and
a first photopolymerized mass transport limiting membrane formed directly upon the first working electrode and overcoating at least the one or more first active areas.

2. The analyte sensor of claim 1, wherein the first photopolymerized mass transport limiting membrane comprises an acrylate polymer or copolymer, a thiol-ene copolymer, or any combination thereof.

3. The analyte sensor of claim 1, further comprising:
a second working electrode;
one or more second active areas disposed upon the second working electrode, the one or more second active areas being responsive to a second analyte different from the first analyte; and
a second mass transport limiting membrane overcoating at least the one or more second active areas and differing in composition from the first photopolymerized mass transport limiting membrane.

4. The analyte sensor of claim 3, wherein the second mass transport limiting membrane is a second photopolymerized mass transport limiting membrane differing in composition from the first photopolymerized mass transport limiting membrane.

5. The analyte sensor of claim 1, wherein the first photopolymerized mass transport limiting membrane is crosslinked.

6. The analyte sensor of claim 1, wherein the first photopolymerized mass transport limiting membrane is discontinuous, optionally wherein the first photopolymerized mass transport limiting membrane is disposed substantially upon the one or more active areas.

7. The analyte sensor of claim 1, wherein the one or more first active areas comprise one or more analyte-responsive enzymes.

8. The analyte sensor of claim 1, wherein the first working electrode is disposed upon a sensor tail configured for insertion in a tissue.

9. A method comprising:
providing an analyte sensor comprising at least a first working electrode having one or more first active areas disposed thereon, the one or more first active areas being responsive to a first analyte;
applying one or more first monomers upon the first working electrode in contact with the one or more first active areas; and
polymerizing the one or more first monomers *in situ* upon the first working electrode to form a first mass transport limiting membrane overcoating at least the one or more first active areas.

10. The method of claim 9, wherein the first mass transport limiting membrane is a photopolymerized mass transport limiting membrane formed by photopolymerizing the one or more first monomers.

11. The method of claim 10, wherein the first photopolymerized mass transport limiting membrane comprises an acrylate polymer or copolymer, a thiol-ene copolymer, or any combination thereof.

12. The method of claim 9, wherein applying the one or more first monomers comprises screen printing the one or more first monomers, spraying the one or more first monomers, or any combination thereof.

13. The method of claim 9, wherein the analyte sensor further comprises a second working electrode having one or more second active areas disposed thereon, the one or more second active areas being responsive to a second analyte different from the first analyte, the method further comprising:
applying one or more second monomers upon the second working electrode in contact with the one or more second active areas; and
polymerizing the one or more second monomers *in situ* upon the second working electrode to form a second mass transport limiting membrane overcoating at least the one or more second active areas, the second mass transport limiting membrane differing in composition from the first mass transport limiting membrane.

14. The method of claim 13, wherein the second mass transport limiting membrane is a photopolymerized mass transport limiting membrane formed by photopolymerizing the one or more second monomers.

15. The method of claim 9, wherein the first mass transport limiting membrane is discontinuous, optionally wherein the first mass transport limiting membrane is formed selectively on the one or more first active areas.
